(19) Europäisches Patentamt
European Patent Office
Office européen des brevets

(11) Veröffentlichungsnummer: **0 320 817**
**A1**

(12) **EUROPÄISCHE PATENTANMELDUNG**

(21) Anmeldenummer: **88120616.3**

(51) Int. Cl.⁴: **C07D 281/08 , A61K 31/55**

(22) Anmeldetag: **09.12.88**

(30) Priorität: **17.12.87 US 134282**

(43) Veröffentlichungstag der Anmeldung: **21.06.89 Patentblatt 89/25**

(84) Benannte Vertragsstaaten: **BE CH DE FR GB IT LI NL SE**

(71) Anmelder: **F. HOFFMANN-LA ROCHE & CO. Aktiengesellschaft**

**CH-4002 Basel(CH)**

(72) Erfinder: **Mohacsi, Erno**
**85 Hillcrest Avenue**
**Summit, N.J. 07901(US)**
Erfinder: **O'Brien, Jay Philip**
**71 Ridge Road**
**Cedar Grove, N.J. 07009(US)**

(74) Vertreter: **Kellenberger, Marcus, Dr. et al**
**Grenzacherstrasse 124 Postfach 3255**
**CH-4002 Basel(CH)**

(54) **2,3-Dihydro-2-phenyl-5-amino-alkyl-naphtho[2,1-b][1,4]thiazepinon-Derivate.**

(57) Verbindungen der Formel

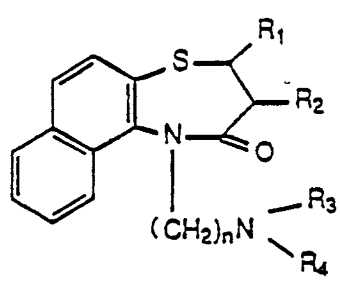

I

worin $R_1$ Phenyl mit 1-3 Niederalkoxy- oder Halogensubstituenten, $R_2$ Hydroxy, Niederalkoxy, Niederalkanoyloxy, Niedercycloalkylcarbonyloxy, -O-COO-($C_1$-$C_5$-Alkyl) oder -O-CO($CH_2$)$_m$-O-($C_1$-$C_3$-Alkyl), $R_3$ und $R_4$ unabhängig voneinander je Niederalkyl oder Phenylniederalkyl oder zusammen einen Piperidin- oder Pyrrolidinring, n 2-4 und m 1 oder 2 bedeuten,

und pharmazeutisch verwendbare Säureadditionssalze davon besitzen den Calciumkanal blockierende Eigenschaften und eignen sich demnach als Mittel zur Behandlung von Ischämie und als Blutdrucksenker.

EP 0 320 817 A1

## 2,3-Dihydro-2-phenyl-5-aminoalkyl-naphtho[2,1-b][1,4]thiazepinon-Derivate

Die vorliegende Erfindung betrifft Naphtho[2,1-b][1,4]thiazepin-4(5H)-one der allgemeinen Formel

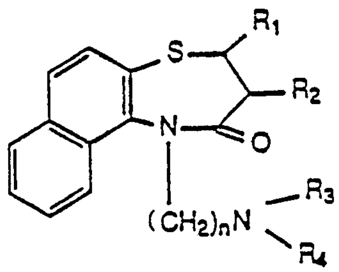

I

worin $R_1$ Phenyl mit 1-3 Niederalkoxy- oder Halogen-substituenten, $R_2$ Hydroxy, Niederalkoxy, Niederalkanoyloxy, Niedercycloalkylcarbonyloxy, -O-COO-($C_1$-$C_5$-Alkyl) oder -O-CO($CH_2$)$_m$-O-($C_1$-$C_3$-Alkyl), $R_3$ und $R_4$ unabhängig voneinander je Niederalkyl oder Phenylniederalkyl oder zusammen einen Piperidin- oder Pyrrolidinring, n 2-4 und m 1 oder 2 bedeuten,
und pharmazeutisch verwendbare Säureadditionssalze davon.

Diese Verbindungen besitzen Calcium-antagonistische Eigenschaften und eignen sich demnach zur Behandlung von Ischämie und Bluthochdruck.

Der in dieser Beschreibung verwendete Ausdruck "Niederalkyl" betrifft geradkettige oder verzweigte Alkylgruppen mit 1-4 Kohlenstoffatomen beispielsweise Methyl, Aethyl, Propyl, Isopropyl, Butyl und dergleichen. Alternativ kann die Anzahl Kohlenstoffatome einer Alkylgruppe angegeben sein, wie im Ausdruck "$C_1$-$C_3$-Alkyl", welcher eine geradkettige oder verzweigte Alkylgruppe mit 1-3 Kohlenstoffatomen betrifft. Der Ausdruck "Niederalkoxy" betrifft geradkettige oder verzweigte Niederalkoxygruppen mit 1-4 Kohlenstoffatomen, beispielsweise Methoxy, Aethoxy, Propoxy, Isopropoxy, Butoxy und dergleichen. Der Ausdruck "Halogen" betrifft die vier Halogene Brom, Chlor, Fluor und Jod. Der Ausdruck "Niederalkanoyloxy" betrifft geradkettige oder verzweigte Alkanoyloxygruppen mit 2-5 Kohlenstoffatomen, beispielsweise Acetyloxy, Propionyloxy, Butyryloxy, Isopropionyloxy und dergleichen. Der Ausdruck "Niedercycloalkyl" betrifft niedere Cycloalkylgruppen mit 3-6 Kohlenstoffatomen, beispielsweise Cyclopropyl, Cyclobutyl, Cyclopentyl und Cyclohexyl. Der Ausdruck "Phenylniederalkyl" betrifft eine durch eine Phenylgruppe substituierte Niederalkylgruppe, beispielsweise Phenylmethyl, Phenyläthyl, Phenylpropyl, Phenylbutyl und dergleichen.

Die in den Formeln der vorliegenden Anmeldung verwendete ausgezogene Linie ( 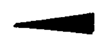 ) bedeutet, dass sich ein Substituent oberhalb der Ebene des schwefel- und stickstoffhaltigen Rings befindet, während eine gestrichelte Linie ( 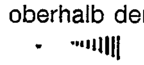 ) bedeutet, dass sich ein Substituent unterhalb der Ebene des schwefel- und stickstoffhaltigen Rings befindet.

Die Verbindungen der Formel I enthalten 2 asymmetrische Zentren in den 2- und 3-Stellungen. Demgemäss können die Verbindungen der Formel I als Stereoisomere auftreten, d.h. als cis- oder trans-Isomere.

Der in dieser Beschreibung verwendete Ausdruck "cis" betrifft eine Verbindung, worin die Substituenten $R_1$ und $R_2$ beide auf derselben Seite der Ebene des schwefel- und stickstoffhaltigen Rings liegen. Der in dieser Beschreibung verwendete Ausdruck "(+)-cis" betrifft ein Enantiomeres, welches eine absolute Konfiguration aufweist, die analog der von (2S,3S)-3-(Acetyloxy)-2,3-dihydro-5-[2-(dimethylamino)äthyl] -2-(4-methoxyphenyl)naphtho[2,1-b][1,4]thiazepin-4(5H)-on ist, welches eine erfindungsgemässe (+)-cis-Verbindung ist.

Eine Verbindung der allgemeinen Formel

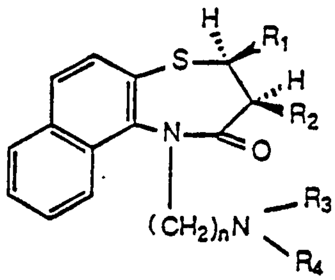

Ia

worin $R_1$, $R_2$, $R_3$, $R_4$ und n die obige Bedeutung besitzen, ist eine erfindungsgemässe (+)-cis-Verbindung.

Eine Verbindung der allgemeinen Formel

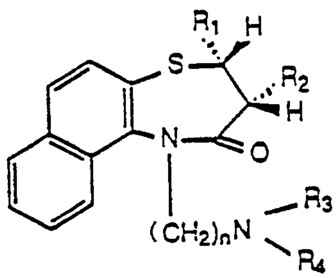

Ib

worin $R_1$, $R_2$, $R_3$, $R_4$ und n die obige Bedeutung besitzen, ist das Enantiomere einer Verbindung der Formel Ia und ist eine erfindungsgemässe (-)-cis-Verbindung.

Bevorzugte Verbindungen sind die cis-Verbindungen.

Speziell bevorzugte Verbindungen sind die (+)-cis-Verbindungen.

Der in dieser Beschreibung verwendete Ausdruck "trans" betrifft eine Verbindung der Formel I, worin die Substituenten $R_1$ und $R_2$ sich auf den gegenüberliegenden Seiten der Ebene des schwefel- und stickstoffhaltigen Rings befinden.

Eine Verbindung der allgemeinen Formel

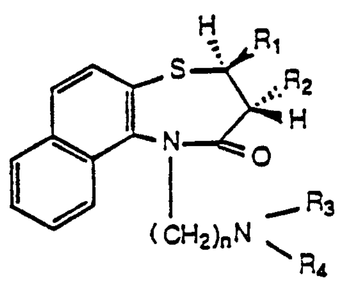

Ic

worin $R_1$, $R_2$, $R_3$, $R_4$ und n die obige Bedeutung besitzen, ist eine erfindungsgemässe trans-Verbindung.

Eine Verbindung der allgemeinen Formel

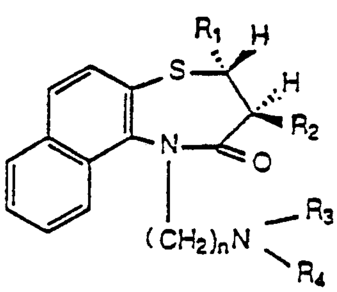

Id

worin $R_1$, $R_2$, $R_3$, $R_4$ und n die obige Bedeutung besitzen,
ist das Enantiomere einer Verbindung der Formel Ic und eine weitere erfindungsgemässe trans-Verbindung.

Bevorzugt sind diejenigen Verbindungen der Formel I, worin $R_1$ 4-Niederalkoxyphenyl und $R_2$ Hydroxy, Niederalkoxy, Niedercycloalkylcarbonyloxy, $-O-CO(CH_2)_m-O-(C_1-C_3\text{-Alkyl})$ oder $-O-CO-O-(C_1-C_5\text{-Alkyl})$ bedeuten. Von diesen Verbindungen sind, wie bereits oben erwähnt, die cis-Verbindungen bevorzugt und die (+)-cis-Verbindungen speziell bevorzugt.

Weitere noch bevorzugtere Verbindungen der Formel I sind solchen worin $R_1$ 4-Niederalkoxyphenyl $R_2$ Niederalkanoyloxy, n 2 oder 3 und $R_3$ und $R_4$ unabhängig voneinander je Niederalkyl bedeuten. Von diesen Verbindungen sind, wie bereits oben erwähnt, die cis-Verbindungen bevorzugt und die (+)-cis-Verbindungen speziell bevorzugt.

Noch bevorzugtere Verbindungen der Formel I sind solche, worin $R_1$ 4-Aethoxyphenyl oder, besonders bevorzugt, 4-Methoxyphenyl, $R_2$ Propionyloxy oder, besonders bevorzugt, Hydroxy oder Acetyloxy, n 2 und $R_3$ und $R_4$ je Aethyl oder, besonders bevorzugt, Methyl bedeuten. Von diesen Verbindungen sind wiederum wie bereits oben erwähnt die cis-Verbindungen bevorzugt und die (+)-cis-Verbindungen speziell bevorzugt.

Beispiele von Verbindungen der Formel I sind:
trans-rac-2,3-Dihydro-3-hydroxy-5-[2-(dimethylamino)äthyl]-2-(4-methoxyphenyl)naphtho[2,1-b][1,4]-thiazepin-4(5H)on;
trans-rac-3-(Acetyloxy) 2,3-dihydro-5-[2-(dimethylamino)äthyl]-2-(4-methoxyphenyl)naphtho[2,1-b][1,4]-thiazepin-4(5H)-on;
cis-(-)-2,3-Dihydro-3-hydroxy-5-[2-(dimethylamino)äthyl]-2-(4-methoxyphenyl)naphtho[2,1-b][1,4]thiazepin-4-(5H)-on;
cis(+)-2,3-Dihydro-3-hydroxy-5-[2-(dimethylamino)äthyl]-2-(4-methoxyphenyl)naphtho[2,1-b][1,4]thiazepin-4-(5H)-on;
cis-(-)-3-(Acetyloxy)-2,3-dihydro-5-[2-(dimethylamino)äthyl]-2-(4-methoxyphenyl)naphtho[2,1-b][1,4]thiazepin-4(5H)-on;
cis-(±)-3-[(Aethoxycarbonyl)oxy]-2,3-dihydro-2-(4-methoxyphenyl)-5-[2-(dimethylamino)äthyl]naphtho[2,1-b]-[1,4]thiazepin-4(5H)-on;
cis-(±)-2,3-Dihydro-3-methoxy-5-[2-(dimethylamino)äthyl]-2-(4-methoxyphenyl)naphtho[2,1-b][1,4]thiazepin-4-(5H)-on;
cis-(±)-2,3-Dihydro-3-(2-methoxy-1-oxoäthoxy)-2-(4-methoxyphenyl)-5-[2-(dimethylamino)äthyl]naphtho[2,1-b][1,4]-thiazepin-4(5H)-on;
cis-(±)-3-[(Cyclopropylcarbonyl)oxy]-2,3-dihydro-5-[2-(dimethylamino)äthyl]-2-(4-methoxyphenyl)naphtho[2,1-b][1,4]-thiazepin-4(5H)-on;
cis-rac-5-[2-(Dimethylamino)propyl]-2,3-dihydro-3-hydroxy-2-(4-methoxyphenyl)naphtho[2,1-b][1,4]thiazepin-4(5H)-on;
cis-rac-3-(Acetyloxy)-2,3-dihydro-2-(4-methoxyphenyl)-5- [3-dimethylamino)propyl]naphtho[2,1-b][1,4]-thiazepin-4(5H)-on:
cis-rac-5-[2-(Diäthylamino)äthyl]-2,3-dihydro-3-hydroxy-2-(4-methoxyphenyl)naphtho[2,1-b][1,4]thiazepin-4-(5H)-on und
cis-rac-3-(Acetyloxy)-5-[2-(diäthylamino)äthyl]-2,3-dihydro-2-(4-methoxyphenyl)naphtho[2,1-b][1,4]thiazepin-4(5H)-on.

Bevorzugtere Verbindungen der Formel I sind:
cis-rac-3-(Acetyloxy)-2,3-dihydro-5-[2-(dimethylamino)äthyl]-2-(4-methoxyphenyl)naphtho[2,1-b][1,4]-thiazepin4(5H)-on und
cis-rac-2,3-Dihydro-3-hydroxy-5-[2-(dimethylamino)äthyl]-2-(4-methoxyphenyl)naphtho[2,1-b][1,4]thiazepin-4-(5H)-on.

Die bevorzugtesten Verbindungen der Formel I sind:
cis-(+)-3-(Acetyloxy)-2,3-dihydro-5-[2-(dimethylamino)äthyl]-2-(4-methoxyphenyl)naphtho[2,1-b][1,4]-thiazepin-4(5H)-on und
cis-(+)-2,3-Dihydro-3 hydroxy-5-[2-(dimethylamino)äthyl]-2-(4-methoxyphenyl)naphtho[2,1-b][1,4]thiazepin-4(5H)-on.

Die Verbindungen der Formel 1 und deren pharmazeutisch verwendbare Säureadditionssalze können erfindungsgemäss hergestellt werden, indem man

a) eine Verbindung der allgemeinen Formel

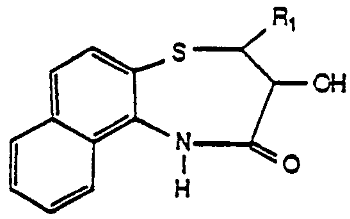

II

worin $R_1$ die obige Bedeutung besitzt, mit einer Verbindung der allgemeinen Formel

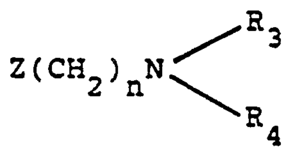

III

worin $R_3$, $R_4$ und n die obige Bedeutung besitzen und Z Halogen bedeutet. umsetzt, und erwünschtenfalls,

b) ein Racemat einer erhaltenen Verbindung der allgemeinen Formel

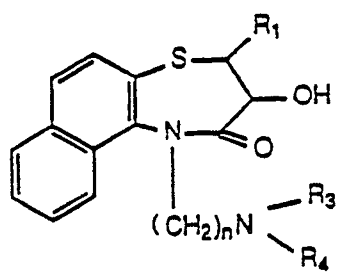

Ie

worin $R_1$, $R_3$, $R_4$ und n die obige Bedeutung besitzen, in die optisch aktiven Enantiomeren auftrennt, und/oder

c) ein Alkalimetallsalz einer Verbindung der Formel Ie mit einem Niederalkylierungsmittel umsetzt, oder

d) eine Verbindung der Formel Ie, mit einem Niederalkansäureanhydrid, einem Niederalkanoylhalogenid, einem $C_1$-$C_5$-Alkylhaloformat, einem $C_1$-$C_3$-Alkoxy-acetyl- oder -propionylhalogenid oder einem Niedercycloalkylcarbonsäurehalogenid umsetzt, und/oder

e) eine erhaltene Verbindung der Formel I in ein pharmazeutisch verwendbares Säureadditionssalz überführt.

Eine Verbindung der obigen Formel II kann in eine Verbindung der allgemeinen Formel

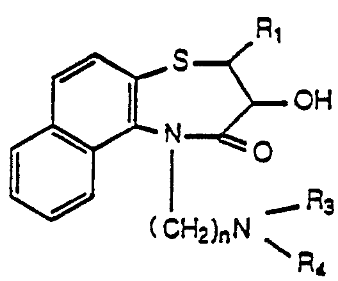

Ie

worin $R_1$, $R_3$, $R_4$ und n die obige Bedeutung besitzen, durch Umsetzen mit einer Verbindung der allgemeinen Formel

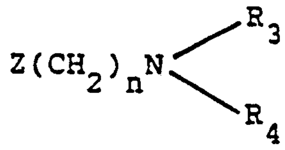

III

worin $R_3$, $R_4$ und n die obige Bedeutung besitzen und Z Halogen, vorzugsweise Chlor, bedeutet, übergeführt werden.

Die Reaktion wird ausgeführt, indem man ein Alkali-metallsalz einer Verbindung der Formel II, wie das Natrium- oder vorzugsweise Kaliumsalz, mit einem Aminoalkylhalogenid der Formel III, vorzugsweise dem Chlorid, in einem polaren organischen Lösungsmittel wie Methylacetat oder bevorzugt Aethylacetat, bei einer Temperatur zwischen ungefähr 40° und 80°C oder bei der Rückflusstemperatur des verwendeten Lösungsmittels, welche im Falle von Aethylacetat 77°C beträgt, während etwa 1-17 Stunden umsetzt. Die Umsetzung erfolgt in Gegenwart einer Base, wie Kaliumhydroxid in Aceton oder bevorzugt Kaliumcarbonat in Aceton oder einem Niederalkylacetat. Die erhaltene Verbindung der Formel Ie kann nach konventionellen Methoden, wie Kristallisation, abgetrennt werden.

Vorgängig der weiter unten beschriebenen Ueberführung einer Verbindung der Formel Ie in andere Verbindungen der Formel I kann eine cis-Verbindung der Formel Ie, welche als Racemat erhalten wird, in die optisch aktiven Enantiomeren aufgetrennt werden. Die Auftrennung einer speziellen cis-Verbindung der Formel Ie, nämlich von cis-rac-2,3-Dihydro-3-hydroxy-5-[2-(dimethylamino)äthyl] -2-(4-methoxyphenyl)-naphtho[2,1-b][1,4]thiazepin-4(5H)-on ist im Formelschema I veranschaulicht. Die Auftrennung anderer Verbindungen der Formel Ie kann beispielsweise andere übliche Mittel zur Auftrennung notwendig machen.

Formelschema I

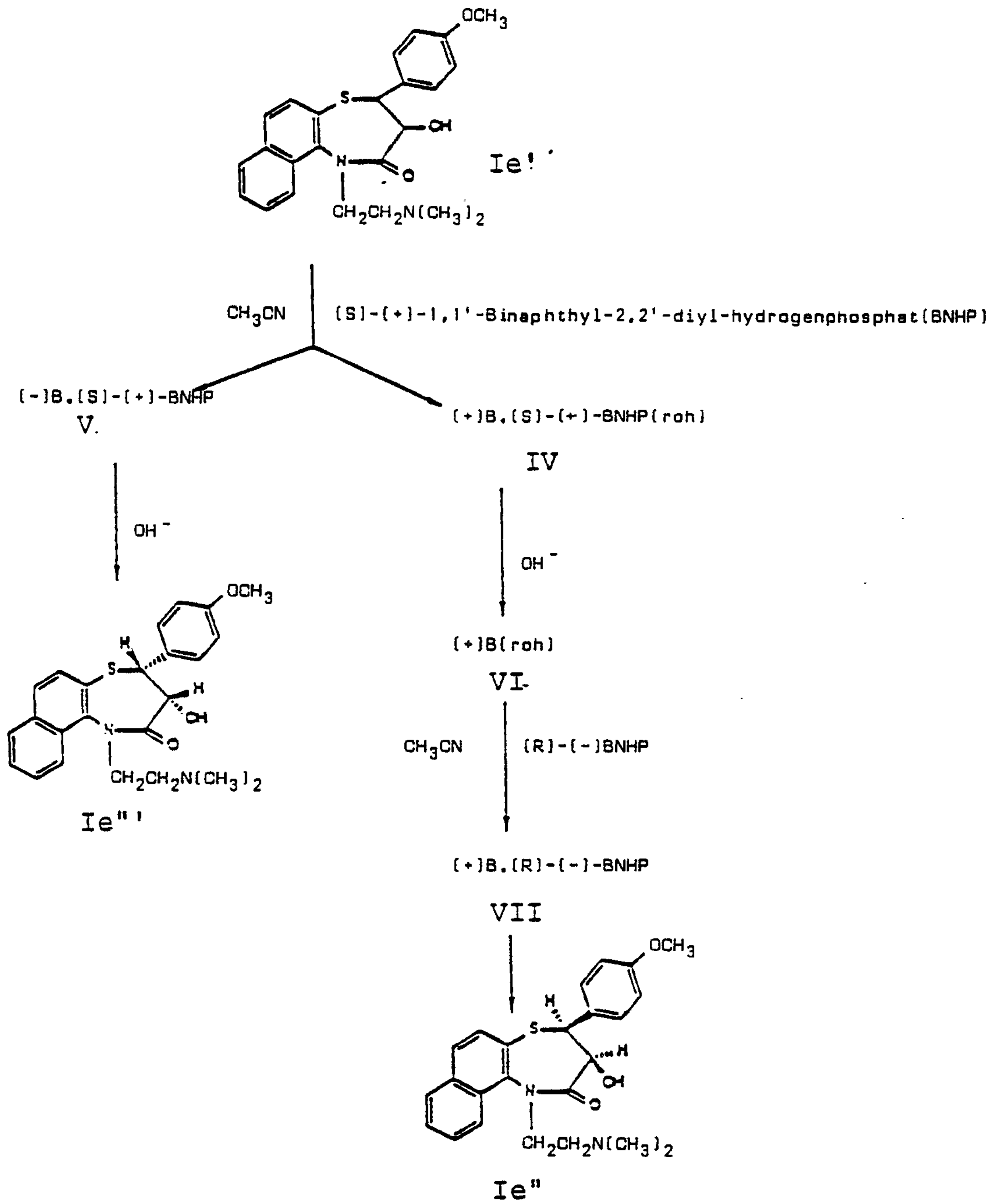


worin (+) -B bzw. (-)-B die (+)- bzw. (-)-Enantiomeren der Verbindung der Formel Ie′, d.h. von cis-rac-2,3-Dihydro--3-hydroxy -5-[2-(dimethylamino)äthyl] -2-(4-methoxyphenyl)naphtho[2,1-b][1,4]thiazepin-4(5H)-on, sind.

Gemäss Formelschema I setzt man ein Racemat der Formel Ie′ in einem polaren, aprotisch organischen Lösungsmittel, wie Acetonitril, mit einer heissen Acetonitril-Lösung eines Spaltungsmittels, wie S-(+)-1,1′-Binaphthyl-2,2′-diyl-hydrogenphosphat (BNHP). um und lässt die erhaltene Lösung bei Raumtemperatur kristallisieren. Die Kristalle bestehen aus einem Salz der Formel V aus dem Spaltungsmittel und dem (-)-Enantiomeren der Verbindung der Formel Ie′. Das lösliche Salz ist dasjenige des (+)-Enantiomeren der Verbindung der Formel Ie′ und des Spaltungsmittels. Dies ist die Lösung der Formel IV im Formelschema I.

Die Kristalle der Formel V werden abfiltriert.

Das Salz der Formel V kann in Wasser mit einer Base, wie Natriumhydroxid oder vorzugsweise konz.

Ammoniumhydroxid, versetzt werden, und die wässrige Suspension mit einem organischen Lösungsmittel, wie Methylenchlorid, extrahiert und anschliessend eingedampft werden, wobei man das (-)-Enantiomere der Formel I‴ erhält. Das (-)-Enantiomere kann in den unten beschriebenen Reaktionen von Verbindungen der Formel Ie eingesetzt werden.

Die oben erwähnte Lösung der Formel IV wird eingedampft und in Wasser mit einer Base, wie Natriumhydroxid oder besonders bevorzugt konz. Ammoniumhydroxid, versetzt und mit einem organischen Lösungsmittel, wie Aether, extrahiert und eingedampft, wobei man das rohe (+)-Enantiomere der Formel VI im Formelschema I erhält. Das rohe (+)-Enantiomere der Formel VI kann weiter gereinigt werden, indem man es in heissem Acetonitril löst und die erhaltene Lösung mit einem Spaltungsmittel, wie R-(-)-1,1′Binaphthyl-2,2′-diyl-hydrogenphosphat in Acetonitril versetzt. Dabei erhält man ein Salz dieses Spaltungsmittels mit dem (+)-Enantiomeren der Formel VII. Dieses Salz kann in Wasser suspendiert werden und mit einer Base, wie konz. Ammoniumhydroxid, versetzt werden, und die erhaltene Suspension mit einem organischen Lösungsmittel, wie Aether, extrahiert werden. Das (+)-Enantiomere der Formel Ie″ kann aus der Lösung durch Abdampfen des Lösungsmittels isoliert werden und in die unten beschriebenen Reaktionen von Verbindungen der Formel Ie eingesetzt werden.

Die trans Racemate der Formel Ie können in ähnlicher Weise unter Verwendung üblicher Spaltungsmittel aufgetrennt werden.

Eine Verbindung der Formel I, worin $R_2$ Niederalkoxy bedeutet, kann hergestellt werden, indem man ein Alkalimetallsalz einer Verbindung der Formel Ie, wie ein Natriumsalz (hergestellt durch Umsetzen einer Verbindung der Formel Ie mit einem Alkalimetallhydrid, wie Natriumhydrid), mit einem Niederalkylierungsmittel, wie einem Dialkylsulfat, insbesondere Dimethylsulfat, in einem aromatischen Lösungsmittel, wie Toluol oder vorzugsweise Benzol, bei ungefähr Rückflusstemperatur etwa 10 Minuten bis etwa 2 Stunden umsetzt. Die erhaltene Verbindung der Formel I kann nach konventionellen Methoden, wie Kristallisation, isoliert werden.

Eine Verbindung der Formel Ie kann acyliert werden, indem man sie mit einem Niederalkansäureanhydrid, wie Propionsäureanhydrid oder Essigsäureanhydrid oder einem Niederalkanoylhalogenid, beispielsweise Acetyl-, Propionyl- oder Butyrylchlorid, gegebenenfalls in Gegenwart einer Base, wie Pyridin, Triäthylamin oder Dimethylanilin, bei Raumtemperatur oder einer Temperatur bis ungefähr 115°C umsetzt.

Alternativ kann eine Verbindung der Formel I, worin $R_2$-O-CO-O-($C_1$-$C_5$-Alkyl) bedeutet, hergestellt werden, indem man eine Verbindung der Formel Ie mit einem $C_1$-$C_5$-Alkylhaloformat, wie Aethylchloroformat, in einem basischen Lösungsmittel, wie Pyridin, bei ungefähr Eisbadtemperatur umsetzt. Die erhaltene Verbindung der Formel I kann nach konventionellen Methoden, wie Kristallisation, isoliert werden.

Eine Verbindung der Formel I, worin $R_2$ -O-CO$(CH_2)_m$-O-($C_1$-$C_3$-Alkyl) bedeutet, kann hergestellt werden, indem man eine Verbindung der Formel Ie mit einem $C_1$-$C_3$-Alkoxyacetyl- oder -propionylhalogenid, wie Methoxyacetylchlorid, in einem basischen Lösungsmittel, wie Pyridin bei ungefähr Eisbadtemperatur umsetzt. Die erhaltene Verbindung der Formel I kann nach konventionellen Methoden, wie Kristallisation, isoliert werden.

Eine Verbindung der Formel I, worin $R_2$ Cycloalkylcarbonyloxy bedeutet, kann hergestellt werden, indem man eine Verbindung der Formel Ie mit einem Cycloalkylcarbonsäurehalogenid, wie Cyclopropancarbonsäurechlorid, in einem basischen Lösungsmittel, wie Pyridin, bei ungefähr Eisbadtemperatur etwa 1-17 Stunden umsetzt. Die erhaltene Verbindung der Formel I kann nach konventionellen Methoden, wie Extraktion, isoliert werden.

Eine Verbindung der Formel I, worin $R_2$ von Hydroxy verschieden ist, kann durch Umsetzen mit einer organischen Säure, wie Essigsäure, Oxalsäure, Malonsäure, Weinsäure, Apfelsäure, Zitronensäure, Milchsäure, Maleinsäure oder Fumarsäure in einem geeigneten organischen Lösungsmittel, wie Aethylacetat, Aceton, Methanol oder Aethanol, in ein entsprechendes Säureadditionssalz übergeführt werden. Alternativ kann eine Verbindung der Formel I, worin $R_2$ von Wasserstoff verschieden ist, in ein entsprechendes Säureadditionssalz übergeführt werden, indem man die Verbindung mit einer anorganischen Säure, wie Schwefelsäure, Bromwasserstoffsäure oder vorzugsweise Chlorwasserstoffsäure, umsetzt, ausgenommen in solchen Fällen, in denen der $R_2$-Substituent durch eine solche Behandlung abgespalten würde. Eine erhaltene Verbindung der Formel I, worin $R_2$ Hydroxy bedeutet, kann in ein entsprechendes Säureadditionssalz übergeführt werden, indem man sie mit einer organischen Säure wie oben beschrieben oder einer anorganischen Säure, wie Chlorwasserstoffsäure, in einem geeigneten organischen Lösungsmittel, wie Aethylacetat, umsetzt.

Die Verbindungen der Formel II können, wie im nachstehenden Formelschema II beschrieben, hergestellt werden. In diesem Formelschema besitzt $R_1$ die obige Bedeutung, bedeutet $R_5$ Niederalkyl und der Stern * ein asymmetrisches Kohlenstoffatom.

## Formelschema II

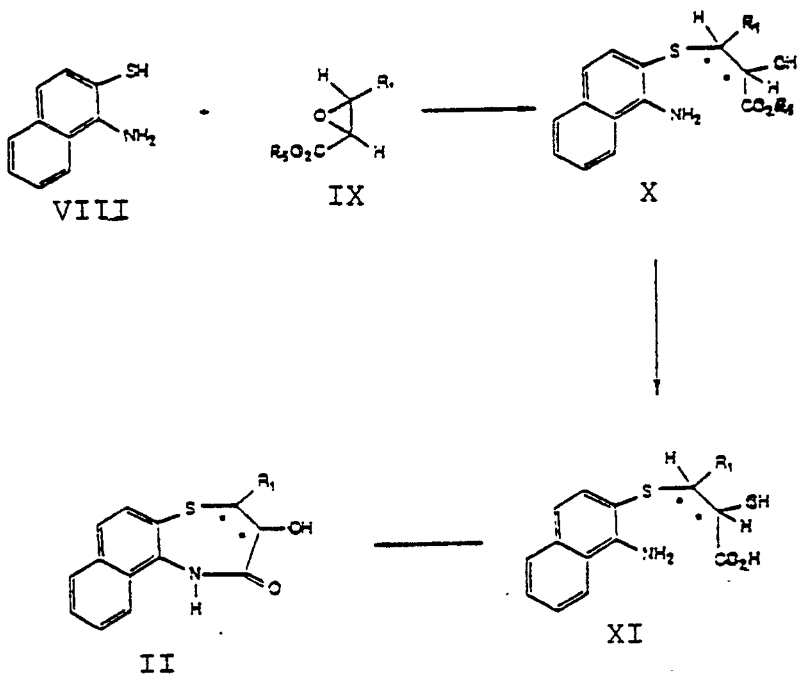

VIII IX X

XI

II

Im Zusammenhang mit der nachstehenden Beschreibung der Reaktionsschritte im Formelschema II ist darauf hinzuweisen dass die Ausdrücke "SR" und "RR" sich auf die relativen Konfigurationen der Hydroxy- und $R_1$-Substituenten in den 2- und 3-Stellungen der Verbindungen der Formeln X und XI beziehen. So betrifft der Ausdruck "SR" Verbindungen, worin sich die Hydroxy- und $R_1$-Substituenten auf der gleichen Seite der Bindung zwischen der 2- und 3-Stellung in der nach Fischer projizierten Formel befinden. Der Ausdruck "RR" betrifft Verbindungen der Formeln X und XI, worin die Hydroxy- und $R_1$-Substituenten sich auf den gegenüberliegenden Seiten der Bindung zwischen der 2- und 3-Stellung der nach Fischer projizierten Formel befinden. Eine Fischer-Projektionsformel einer "SR" Verbindung der Formel I wird wie folgt dargestellt

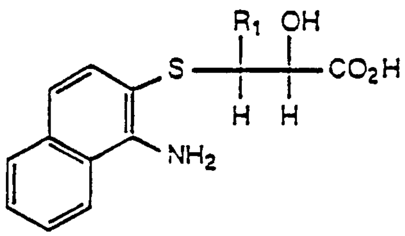

worin $R_1$ die obige Bedeutung besitzt.

Eine Fischer-Projektionsformel einer "RR" Verbindung der Formel XI präsentiert sich wie folgt

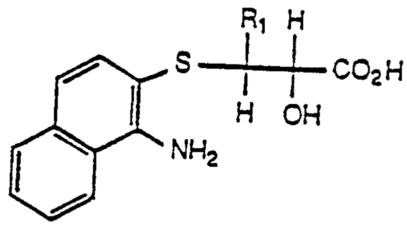

worin $R_1$ die obige Bedeutung besitzt.

In der nachstehenden Beschreibung der Reaktionsschritte wird der Ester der Formel X als Gemisch von "SR" und "RR" Isomeren erhalten, welche durch fraktionierte Kristallisation getrennt werden.

Die "SR" Verbindungen der Formeln X und XI werden in Form von Racematen erhalten und werden in dieser Form weiter umgesetzt. Die "RR" Verbindungen der Formeln X und XI werden ebenfalls in Form von Racematen erhalten und als solche weiter umgesetzt.

Gemäss Formelschema II wird 1-Aminonaphthalin-2-thiol der Formel VIII mit einer Verbindung der allgemeinen Formel

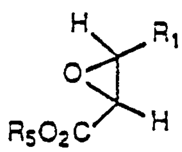

IX

worin $R_1$ und $R_5$ die obige Bedeutung besitzen,
zu einer Verbindung der allgemeinen Formel

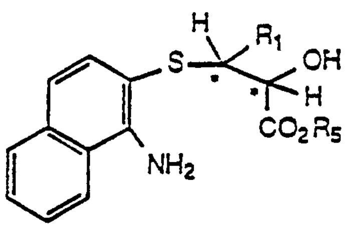

X

worin $R_1$, $R_5$ und der Stern * die obige Bedeutung besitzen,
umgesetzt.

Die Umsetzung erfolgt in Abwesenheit eines Lösungsmittels oder in Gegenwart eines aromaatischen Lösungsmittels, wie Benzol, Toluol, Xylol oder Aethylbenzol, oder Acetonitril in Gegenwart einer katalytischen Menge von Kaliumcarbonat bei einer Temperatur zwischen etwa 80° und 140° C unter einer Argon- oder vorzugsweise Stickstoffatmosphäre während 1-20 Stunden. Das molare Verhältnis der Reaktanten ist nicht kritisch. Vorzugsweise werden ]edoch die Reaktanten in einem 1:1-Verhältnis eingesetzt.

Falls die Reaktion in einem nicht-polaren, organischen Lösungsmittel, vorzugsweise Toluol, durchgeführt wird, erhält man ein Gemisch der "SR" und "RR" Verbindungen der Formel X. Ein solches Gemisch kann aufgetrennt werden, indem man dieses mit einer anorganischen waserfreien Säure, wie wasserfreiem Chlorwasserstoff, in einem polaren organischen Lösungsmittel, wie Aethylacetat, umsetzt, wobei man das Hydrochloridsalz einer "SR" Verbindung erhält. Dieses Hydrochloridsalz einer "SR" Verbindung kann mit einer Base, wie Kaliumhydroxid oder vorzugsweise Natriumhydroxid, behandelt werden, wobei man eine "SR" Verbindung erhält.

Falls die Umsetzung in Acetonitril in Gegenwart einer katalytischen Menge der Base Kaliumcarbonat durchgeführt wird, erhält man eine "RR" Verbindung der Formel X. Eine "RR" Verbindung der Formel X kann isoliert werden, indem man das Reaktionsgemisch chromatographiert und nachfolgend umkristallisiert.

Eine "SR" oder "RR" Verbindung der Formel X kann zur entsprechenden "SR" oder "RR" Verbindung der allgemeinen Formel

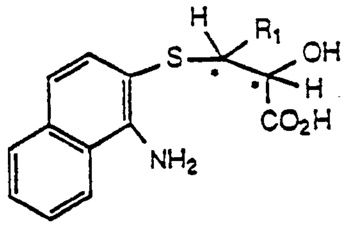

XI

worin $R_1$ und der Stern * die obige Bedeutung besitzen, nach üblichen Hydrolysemethoden hydrolysiert werden. Beispielsweise kann man die Verbindung der Formel X mit einer anorganischen Säure, wie Chlorwasserstoffsäure oder Schwefelsäure, oder mit einer Alkalibase, wie Kaliumhydroxid oder vorzugsweise Natriumhydroxid, behandeln. Diese Umsetzung erfolgt in einem polaren organischen Lösungsmittel, wie einem Alkanol, beispielsweise Propanol oder vorzugsweise Aethanol, bei der Rückflusstemperatur während etwa 10 Minuten bis eine Stunde. Das Produkt kann nach üblichen Methoden, wie Kristallisation, abgetrennt werden.

Eine "SR" Verbindung der Formel XI kann zu einem Racemat der allgemeinen Formel

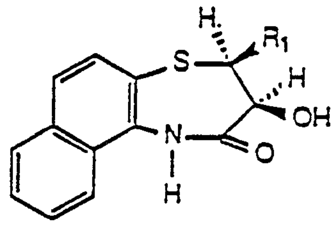

II'

worin $R_1$ die obige Bedeutung besitzt, cyclisiert werden, indem man diese Verbindung in Gegenwart einer katalytischen Menge einer Säure, wie P-Toluolsulfonsäure, in einem aromatischen Lösungsmittel, wie Benzol, Xylol oder vorzugsweise Toluol, während etwa 12-72 Stunden zum Rückfluss erhitzt. Die Verbindung der Formel II′ kann nach üblichen Methoden, wie Umkristallisation, abgetrennt werden.

Das "RR" Racemat der Formel XI kann zu einem Racemat der allgemeinen Formel

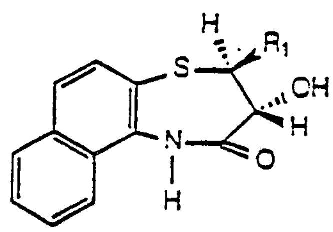

II"

worin $R_1$ die obige Bedeutung besitzt, cyclisiert werden, indem man diese in einer wässrigen anorganischen Säure, wie wässrige Schwefelsäure, zum Rückfluss erhitzt. Eine Verbindung der Formel II″ kann nach üblichen Methoden, wie Umkristallisation, abgetrennt werden.

In den oben beschriebenen Reaktionen bedeutet die Formel II entweder ein cis-Racemat der Formel II′ oder ein trans -Racemat der Formel II″.

Verwendet man eine Verbindung der Formel II′ in den oben beschriebenen Reaktionen, so erhält man eine cis-Verbindung der Formel I.

Verwendet man hingegen eine Verbindung der Formel II″ in den oben beschriebenen Reaktionen, so erhält man eine trans-Verbindung der Formel I.

Die Verbindung der Formel VIII, d.h. 1-Aminonaphthalin-2-thiol, kann wie folgt hergestellt werden. Zu einer Lösung einer Base, wie Kaliumhydroxid oder vorzugsweise Natriumhydroxid, und einem Alkylenglycol, wie Aethylenglycol, wird das bekannte Naphto[1,2-d]thiazol-2-amin, welches nach bekannten Methoden hergestellt werden kann, zugegeben. Das Reaktionsgemisch wird unter einer Inertgasatmosphäre, wie

Stickstoff, während etwa 10-25 Stunden zum Rückfluss erhitzt, danach mit Wasser verdünnt und mit Essigsäure neutralisiert. Die wässrige Suspension wird mit Aether extrahiert, und das Lösungsmittel entfernt. Die Verbindung der Formel VIII, d.h. das 1-Amino-naphthalin-2-thiol, kann aus dem Rückstand nach konventionellen Methoden erhalten werden.

Die Verbindungen der Formel IX sind bekannt oder können nach bekannten Methoden hergestellt werden. Beispiele von Verbindungen der Formel IX sind:

trans-3-(p-Methoxyphenyl)glycidat und
trans-3-(p-Aethoxyphenyl)glycidat.

Die Verbindungen der Formel III sind bekannt oder können nach bekannten Methoden hergestellt werden. Beispiele von Verbindungen der Formel III sind:

2-Dimethylaminoäthylchlorid,
2-Dimethylaminoäthylbromid,
2-Diäthylaminoäthylchlorid,
2-Dipropylaminoäthylchlorid und
3-Dimethylaminopropylchlorid.

Die Verbindungen der Formel I, einschliesslich der verwendbaren Säureadditionssalze davon sind Calciumantagonisten, insbesondere Calciumkanalblocker, und eignen sich demnach zur Blutdrucksenkung und als Mittel zur Behandlung von Ischämie. Deren wertvolle pharmakologische Wirkung kann an Warmblütern im nachstehend beschriebenen Standardtest gezeigt werden.

<u>Meerschweinchen Ileumtest - Tonische Kontraktion</u>

Man betäubt männliche Meerschweinchen mit einem Gewicht von 300-400 g und lässt sie ausbluten. Der Unterbauch wird geöffnet und 10-15 cm des terminalen Ileums werden vorsichtig entfernt und gereinigt und in Tyrodelösung folgender Zusammensetzung gelegt: Natriumchlorid (8 g/l), Kaliumchlorid (0,2 g/l). Magnesiumchlorid (0,2 g/l), Calciumchlorid (0,2 g/l). Natriumhydrogenphosphat (0,05 g/l), Natriumbicarbonat (1,0 g/l) und Glukose (1 g/l). Die Lösung wird bei 37 °C gehalten und mit 95% Stickstoff und 5% Kohlendioxid begast. Man stülpt das Ileum in Abschnitten über einen Glasstab und bringt zur Trennung der äusseren Langsmuskelschicht der Länge des Mesenteralgewebes entlang einen oberflachlichen Schnitt an. Der Längsmuskel wird von den darunterliegenden Circularmuskeln vorsichtig abgetrennt (H.P. Rang , Annals of N.Y. Academy of Science, Vol. 144, Seite 756, (1964)). Das Gewebe wird am einen Ende an einen Gewebehalter fixiert, und das andere Ende mit einem Faden mit einem Krafttransducer verbunden und in einem Tyrodelösung enthaltenden Muskelbad suspendiert, welches bei 37° gehalten wird und mit 95% Sauerstoff und 5% Kohlendioxid begast wird. Man verwendet einen Anfangsdruck von 500 mg und lässt das Gewebe während 60 Minuten vor dem Start des Versuchs sich äquilibrieren. Während dieser Periode wird das Gewebe alle 16 Minuten gewaschen. Jedes Präparat wird in 16-minütigem Intervall mit soviel Kaliumchlorid versetzt, dass man während 2 Minuten eine Kaliumionenkonzentration im Bad von 80 mMK erhält, und danach mit frischer Lösung gewaschen. Das 16-minütige Intervall zwischen den Kaliumionenzugaben wird durch den ganzen Versuch beibehalten. Nach Stabilisierung der Antworten auf die Kaliumionenzugabe wird die Versuchsverbindung (potentieller Calciumantagonist) 2 Minuten vor und während der 2-minütigen Kaliumionenzugabe zum Bad gegeben, und danach das Bad geklärt und mit frischer Lösung gewaschen. Logarithmisch steigende Dosen (bis zu $10^{-4}$ Mol) des potentiellen Antagonisten werden in dem Versuch eingesetzt.

Das Mass, mit welchem eine Verbindung die tonische Muskelkontraktion hemmt, ist das Mass ihrer den Calciumkanal blockierenden Aktivität. Der $IC_{50}$ -Wert ist diejenige Konzentration, bei welcher eine Verbindung die tonische Muskelkontraktion um 50% hemmt.

Die Aktivität von erfindungsgemässen Verbindungen in diesem Test sind in Tabelle I zusammengefasst:

Tabelle I

| Verbindungen | Meerschweinchen-Ileum-Test - Tonische Kontraktion $IC_{50}$ ( M) |
|---|---|
| A | $4{,}0 \times 10^{-7}$ |
| B | $4{,}5 \times 10^{-7}$ |
| C | $2{,}3 \times 10^{-7}$ |
| D | $3{,}9 \times 10^{-7}$ |
| E | $2{,}5 \times 10^{-7}$ |
| Verbindung A: cis-rac-2,3-Dihydro-3-hydroxy-5-[2-(dimethylamino)äthyl]-2-(4-methoxyphenyl)naphtho[2,1-b][1,4]thiazepin-4(5H)-on-hydrochlorid, Verbindung B: cis-rac-3-(Acetyloxy)-2,3-dihydro-5-[2-(dimethylamino)äthyl]-2-(4-methoxyphenyl)naphtho[2,1-b]-[1,4]thiazepin-4(5H)-on-(E)-2-butendioat, Verbindung C: cis-(+)-2,3-Dihydro-3-hydroxy-5-[2-(dimethylamino)äthyl]-2-(4-methoxyphenyl)naphtho[2,1-b][1,4]thiazepin-4(5H)-on-hydrochlorid-semihydrat, Verbindung D: cis-(+)-3-(Acetyloxy)-2,3-dihydro-5-[2-(dimethylamino)äthyl]-2-(4-methoxyphenyl)naphtho[2,1-b]-[1,4]thiazepin-4(5H)-on-(E)-2-butendioat und Verbindung E: Diltiazem hydrochlorid. | |

Die Verbindungen der Formel I und ihre pharmazeutisch verwendbaren Säureadditionssalze können in pharmazeutische Standarddosierungsformen eingearbeitet werden. Die Verbindungen der Formel I eignen sich zur oralen oder parenteralen Applikation mit den üblichen pharmazeutischen Trägermaterialien, beispielsweise organischen oder anorganischen inerten Trägermaterialien, wie Wasser, Gelatine, Lactose, Stärke Magnesiumstearat, Talk, pflanzliche Oele, Gummi, Polyalkylenglycole und dergleichen. Die pharmazeutischen Präparate können in fester Form, z.B. als Tabletten oder Kapseln, oder in flüssiger Form, z.B. als Lösungen, Suspensionen oder Emulsionen, verwendet werden. Pharmazeutische Hilfsstoffe wie Konservierungsmittel, Stabilisierungsmittel, Netzmittel oder Emulgiermittel, Salze zur Aenderung des osmotischen Druckes oder Puffer können zugegeben werden. Die pharmazeutischen Präparate können auch noch andere therapeutisch aktive Stoffe enthalten.

Die Erfindung betrifft auch eine Methode zur Induzierung von Calcium-antagonistischer Wirkung in Warmblütlern, die einer solchen Behandlung bedürfen, welche darin bestehet, dass man eine wirksame Menge einer Verbindung der Formel I verabreicht. Die Erfindung betrifft auch eine Methode zur Senkung des Bluthochdrucks oder zur Behandlung von Ischämie durch myocardialen Schutz während der Ischämie, welche darin bestehet, dass man eine wirksame Dosis einer Verbindung der Formel I oder eines pharmazeutisch verwendbaren Salzes davon einem Warmblütler verabreicht, der einer solchen Behandlung bedarf. Die Menge einer täglichen oralen Dosis kann durch den Fachmann bestimmt werden und ist mit derjenigen von Diltiazem vergleichbar. Die Menge einer intravenösen Dosis kann ebenfalls durch den Fachmann bestimmt werden und ist mit derjenigen von Diltiazem vergleichbar. Es ist jedoch darauf hinzuweisen, dass die Dosis von Individuum zu Individuum variieren kann, weshalb die obige Angabe den Rahmen der vorliegenden Erfindung in keiner Weise beschränken soll.

Die folgenden Beispiele illustrieren die Erfindung. Alle Temperaturen sind in °C angegeben, sofern nichts anderes erwähnt ist.

## Beispiel 1

Naphtho[1,2-d]thiazol-2-amin

Zu 160 ml Thionylchlorid werden 64,0 g (0,32 Mol) 1-(1-Naphthyl)-2-thioharnstoff portionsweise gegeben, während die Temperatur des Reaktionsgemisches bei 30-40° Innentemperatur gehalten wird. Nach beendeter Zugabe werden nochmals 80 ml Thionylchlorid zugegeben, und das Reaktionsgemisch 4 Stunden auf 50-55° erhitzt. Danach kühlt man auf Raumtemperatur ab und verdünnt mit 400 ml Aethylacetat und filtriert. Das Filtrat in 400 ml Wasser wird mit konzentrierter Ammoniumhydroxidlösung basisch gestellt, und die wässrige Suspension mit dreimal 200 ml Aethylacetat extrahiert. Die vereinigten Aethylacetatextrakte werden über Magnesiumsulfat getrocknet, und das Lösungsmittel unter vermindertem Druck abgedampft, wobei man 46,6 g (74%) Naphtho[1,2-d]thiazol-2-amin erhält, Smp. 186-188°.

Beispiel 2

1-Aminonaphthalin-2-thiol

Zu einer Lösung von 30,0 g Natriumhydroxid in 30 ml Wasser und 180 ml Aethylenglycol werden 18,0 g (0,089 Mol) Naphtho[1,2-d]thiazol-2-amin gegeben. Das Reaktionsgemisch wird unter einer Stickstoffatmosphäre während 20 Stunden gerührt und zum Rückfluss erhitzt und danach mit 100 ml Wasser verdünnt. Nach dem Abkühlen auf Raumtemperatur wird das Reaktionsgemisch mit viermal 95 ml Aether extrahiert. Die wässrige Lösung wird in einem Eisbad gekühlt und mit Essigsäure neutralisiert. Die wässrige Suspension wird mit dreimal 175 ml Aether extrahiert. Die vereinigten Aetherextrakte werden mit Wasser gewaschen und über Magnesiumsulfat getrocknet. Entfernen des Lösungsmittels liefert einen Rückstand, zu welchem Heptan (3 x 50 ml) gegeben wird. Abdestillieren des Lösungsmittels unter vermindertem Druck liefert 10,5 g (67%) 1-Aminonaphthalin-2-thiol. Diese Verbindung ist luftempfindlich, weshalb sie sofort mit dem Glycidat kondensiert wird. Für die Analyse wird eine Probe dieser Verbindung bei 110-115°/6,5 Pa destilliert, Smp. 36-37°.

| $C_{10}H_9NS$(175,18) | | | |
|---|---|---|---|
| Berechnet: | C, 68,56; | H, 5,18; | N, 8,00% |
| Gefunden: | C, 68,78; | H, 5,12; | N, 7,96%. |

Beispiel 3

(±)-(S*,R*)- und (R*,R*)-ß-[(1-Amino-2-naphthalinyl)thio]-α-hydroxy-4-methoxybenzolpropansäuremethylester

Unter Stickstoff wird ein Gemisch von 8,5 g (0,048 Mol) 1-Aminonaphthalin-2-thiol und 10,1 g (0,048 Mol) trans-3-(p-Methoxyphenyl)glycidat in 150 ml Toluol 2 Stunden auf 120° erhitzt. Das Reaktionsgemisch wird auf ein kleineres Volumen eingeengt, die Kristalle abfiltriert, danach mit Aether gewaschen und getrocknet, wobei man 8,75 g (47%) von im Wesentlichen reinen (±)-(S*,R*)-ß-[(1-Amino-2-naphthalinyl)thio]-α-hydroxy-4-methoxybenzolpropansäuremethylester erhält, Smp. 132-134°.

Gaschromatographische Analyse des Rohprodukts zeigt ein 24:1 Verhältnis des (S*,R*):(R*,R*) Ester an.

| $C_{21}H_{21}NO_4S$(383,46) | | | |
|---|---|---|---|
| Berechnet: | C, 65,78; | H, 5,52; | N, 3,65% |
| Gefunden: | C, 65,46; | H, 5,49; | N, 3,65%. |

## Beispiel 4

(±)-(S*,R*)-ß-[(1-Amino-2-naphthalinyl)thio]-α-hydroxy-4-methoxybenzolpropansäuremethylester-hydrochlorid

Eine Probe der (S*,R*)-Base von Beispiel 3 liefert nach Umsetzen mit wasserfreiem Chlorwasserstoff in Acetonitril das rohe Hydrochlorid, welches nach Umkristallisation aus Methanol/Aether (±)-(S*,R*)-ß-[(1-Amino-2-naphthalinyl)thio]-α-hydroxy-4-methoxybenzolpropansäuremethylester-hydrochlorid liefert, Smp. 166-168°.

| $C_{21}H_{21}NO_4S•HCl$(419,92) | | | |
|---|---|---|---|
| Berechnet:<br>Gefunden : | C, 60,07;<br>C, 59,96; | H, 5,28;<br>H, 5,45; | N, 3,34%<br>N, 3,48%. |

## Beispiel 5

Basen katalysierte Reaktion von 1-Aminonaphthalin-2-thiol mit trans-3-(p-Methoxyphenyl)glycidat

Ein Gemisch von 4,1 g (0,023 Mol) 1-Aminonaphthalin-2-thiol und 5,0 g (0,024 Mol) trans-3-(p-Methoxyphenyl)glycidat und 0,5 g Kaliumcarbonat in 80 ml Acetonitril wird unter Stickstoff 2 Stunden gerührt und zum Rückfluss erhitzt. Nach dem Abkühlen auf Raumtemperatur wird das Reaktionsgemisch filtriert, und das Filtrat zur Trockene eingedampft, wobei man 8,7 g eines Gemisches von racemischem (R*,R*)-ß-[(1-Amino-2-naphthalinyl)thio]-α-hydroxy-4-methoxybenzolpropansäuremethylester, 1H-Naphtho-[2,1-b][1,4]thiazin-2(3H)-on und p-Methoxybenzaldehyd erhält. Das Gemisch wird in Methylenchlorid gelöst und an einer Säule von 120 g Silicagel chromatographiert. Die Kolonne wird mit 75 ml-Portionen von Methylenchlorid eluiert. Die Fraktionen 3-9 werden gesammelt, und das Lösungsmittel unter vermindertem Druck entfernt, wobei man 0,6 g eines Oels erhält, dessen NMR-Spektrum mit demjenigen von p-Methoxybenzaldehyd identisch ist. Die Fraktionen 13-18 liefern 1,40 g (28%) 1H-Naphtho[2,1-b][1,4]thiazin-2(3H)-on. Für die Analyse wird eine Probe dieser Verbindung aus Aceton kristallisiert, Smp. 195-196°.

| $C_{12}H_9NOS$ (215,27) | | | |
|---|---|---|---|
| Berechnet:<br>Gefunden : | C, 66,95;<br>C, 66,97; | H, 4,21;<br>H, 4,24; | N, 6,51%<br>N, 6,48%. |

Weiteres Eluieren der Kolonne mit demselben Lösungsmittel (Fraktionen 25-40) liefert 1,0 g (11%) rac-(R*,R*)-ß-[(1-Amino-2-naphthalinyl)thio]-α-hydroxy-4-methoxybenzolpropansäuremethylester, Smp. 125-126°. Eine Analysenprobe wird aus Aether umkristallisiert, Smp. 125-126°.

| $C_{21}H_{21}NO_4S$ (383,46) | | | |
|---|---|---|---|
| Berechnet:<br>Gefunden : | C, 65,78;<br>C, 65,65; | H, 5,52;<br>H, 5,58; | N, 3,65%<br>N, 3,94%. |

## Beispiel 6

(±)-(S*,R*)-ß-[(1-Amino-2-naphthalinyl)thio]-α-hydroxy-4-methoxybenzolpropansäure

Ein Gemisch von 8,9 g (0,023 mol) (±)-(S*,R*)-ß-[(1-Amino-2-naphthalinyl)thio]-α-hydroxy-4-methoxybenzolpropansäuremethylester, 50 ml Aethanol und 140 ml 1N Natriumhydroxidlösung wird 30 Minuten zum Rückfluss erhitzt. Das Reaktionsgemisch wird auf Raumtemperatur abgekühlt, mit 50 ml Wasser verdünnt und mit zweimal 75 ml Aether extrahiert. Die wässrige Lösung wird mit Eis abgekühlt, danach mit Essigsäure neutralisiert und mit dreimal 75 ml Aethylacetat extrahiert. Die vereinigten Extrakte werden mit 75 ml Wasser gewaschen und über Magnesiumsulfat getrocknet. Entfernen des Lösungsmittels liefert 8,2 g (96%) (±)-(S*,R*)-ß-[(1-Amino-2-naphthalinyl)thio]-α-hydroxy-4-methoxybenzolpropansäure, Smp. 167-168°. Eine Analysenprobe wird aus Aethylacetat umkristallisiert, Smp. 167-168°.

| $C_{20}H_{19}NO_4S$ (369,43) | | | |
|---|---|---|---|
| Berechnet: | C, 65,02; | H, 5,18; | N, 3,79% |
| Gefunden : | C, 64,88; | H, 5,10; | N, 3,77%. |

## Beispiel 7

(±)-(R*,R*)-ß-[(1-Amino-2-naphthalinyl)thio]-α-hydroxy-4-methoxybenzolpropansäure

Ein Gemisch von 6,6 g (0,017 Mol) (±)-(R*,R*)-ß-[(1-Amino-2-naphthalinyl)thio]-α-hydroxy-4-methoxybenzolpropansäuremethylester, 45 ml Aethanol und 100 ml 1N Natriumhydroxidlösung wird 30 Minuten zum Rückfluss erhitzt. Das Reaktionsgemisch wird auf Raumtemperatur abgekühlt, mit 50 ml Wasser verdünnt und mit Aether extrahiert. Die wässrige Lösung wird mit Eis gekühlt, danach mit Essigsäure neutralisiert und mit dreimal 60 ml Aethylacetat extrahiert. Die vereinigten Extrakte werden mit 50 ml Wasser gewaschen und über Magnesiumsulfat getrocknet. Entfernen des Lösungsmittels liefert 5,5 g rohes (±)-(R*,R*)-ß-[(1-Amino-2-naphthalinyl)thio]-α-hydroxy-4-methoxybenzolpropansäure, welche in Methylenchlorid gelöst und an einer Säule von 55 g Kieselgel chromatographiert wird. Die Kolonne wird mit 50 ml-Portionen Methylenchlorid eluiert. Die Fraktionen 12-17 werden gesammelt, und das Lösungsmittel unter vermindertem Druck entfernt, wobei man 1,3 g (20%) (±)-(R*,R*)-ß-[(1-Amino-2-naphthalinyl)thio]-α-hydroxy-4-methoxybenzolpropansäure erhält. Eine Analysenprobe wird aus Aether umkristallisiert, Smp. 160-161°.

| $C_{20}H_{19}NO_4S$ (369,43) | | | |
|---|---|---|---|
| Berechnet: | C, 65,02; | H, 5,18; | N, 3,79% |
| Gefunden : | C, 64,83; | H, 5,52; | N, 3,81%. |

## Beispiel 8

Cyclisation von (±)-(R*,R*)-β-[(1-Amino-2-napthalinyl)thio]-α-hydroxy-4-methoxybenzolpropansäure

1,0 g (0,003 Mol) (±)-(R*,R*)-β-[(1-Amino-2-naphthalinyl)thio]-α-hydroxy-4-methoxybenzolpropansäure wird in Aceton gelöst und mit Chlorwasserstoff angesäuert. Das Lösungsmittel wird unter vermindertem Druck abgedampft, und der Rückstand in 100 ml 20%iger Schwefelsäure gelöst und 2 Stunden zum Rückfluss erhitzt. Das Reaktionsgemisch wird auf Raumtemperatur abgekühlt, und das (±)-trans-2,3-Dihydro-3-hydroxy-2-(4-methoxyphenyl)naphtho[2,1-b][1,4]thiazepin-4(5H)-on abfiltriert, wobei man 0,69 g (66%) der eben genannten Verbindung erhält. Eine Analysenprobe wird aus Acetonitril umkristallisiert, Smp. 300-301°.

| $C_{20}H_{17}NO_3S$ (351,42) | | | |
|---|---|---|---|
| Berechnet: | C, 68,36; | H, 4,88; | N, 3,99% |
| Gefunden : | C, 68,21; | H, 4,99; | N, 4,51%. |

## Beispiel 9

(±)-cis-2,3-Dihydro-3-hydroxy-2-(4-methoxyphenyl)naphtho[2,1-b][1,4]thiazepin-4(5H)-on

Ein Gemisch von 2,0 g (0,0054 Mol) (±)-(S*,R*)-ß-[(1-Amino-2-naphthalinyl)thio]-α-hydroxy-4-methoxybenzolpropansäure und einigen wenigen Kristallen P-Toluolsulfonsäure in 150 ml Toluol wird während 17 Stunden zum Rückfluss erhitzt. Der grösste Teil des Lösungsmittels wird unter vermindertem Druck abgedampft, und die Kristalle abfiltriert, wobei man 1,5 g (79%) (±)-cis-2,3-Dihydro-3-hydroxy-2-(4-methoxyphenyl)naphtho[2,1-b][1,4]thiazepin-4(5H)-on erhält, Smp. 255-256°. Eine Analysenprobe wird aus Methylenchlorid/Methanol umkristallisiert, Smp. 255-256°.

| $C_{20}H_{17}NO_3S$ (351,42) | | | |
|---|---|---|---|
| Berechnet: | C, 68,36; | H, 4,88; | N, 3,99% |
| Gefunden : | C, 68,15; | H, 4,89; | N, 3,75%. |

## Beispiel 10

(±)-cis-2,3-Dihydro-3-hydroxy-5-[2-(dimethylamino)äthyl]-2-(4-methoxyphenyl)naphtho[2,1-b][1,4]thiazepin-4-(5H)-on

Ein Gemisch von 4,4 g (0,013 Mol) (±)-cis-2,3-Dihydro-3-hydroxy-2-(4-methoxyphenyl)naphtho[2,1-b]-[1,4]thiazepin-4(5H)-on, 2,0 g pulverisiertem Kaliumcarbonat und 1,6 g (0,0132 Mol) 2-Dimethylaminoäthylchlorid in 100 ml Aethylacetat wird unter Rühren 2 Stunden zum Rückfluss erhitzt. Danach werden in jeweils 2-stündigen Intervallen dreimal je 0,4 g zusätzliches 2-Dimethylaminoäthylchlorid zugegeben. Das Reaktionsgemisch wird insgesamt während 12 Stunden zum Rückfluss erhitzt, danach auf Raumtemperatur abgekühlt und filtriert. Das Filtrat wird mit 150 ml Aethylacetat verdünnt und mit 100 ml Wasser gewaschen. Die Aethylacetatlösung wird über Magnesiumsulfat getrocknet, und das Lösungsmittel entfernt, wobei man 3,6 g (68%) (±)-cis-2,3-Dihydro-3-hydroxy-5-[2-(dimethylamino)äthyl]-2-(4-methoxyphenyl)naphtho[2,1-b]-[1,4]thiazepin-4(5H)-on erhält, Smp. 128-130°.

| $C_{24}H_{26}N_2O_3S$ (442,54) | | | |
|---|---|---|---|
| Berechnet: | C, 68,22; | H, 6,20; | N, 6,63% |
| Gefunden : | C, 68,17; | H, 6,29; | N, 6,44%. |

## Beispiel 11

(±)-cis-2,3-Dihydro-3-hydroxy-5-[2-(dimethylamino)äthyl]-2-(4-methoxyphenyl)naphtho[2,1-b][1,4]thiazepin-4-(5H)-on-hydrochlorid

Eine Probe des Endprodukts von Beispiel 10 liefert nach Umsetzen mit wasserfreiem Chlorwasserstoff in Aceton Hydrochlorid, welches nach Umkristallisation aus Methyläthylketon (±)-cis-2,3-Dihydro-3-hydroxy-5-[2-(dimethylamino)äthyl]-2-(4-methoxyphenyl)naphtho[2,1-b][1,4]thiazepin-4(5H)-on-hydrochlorid, Smp. 155-156°.

| $C_{24}H_{26}N_2O_3S \bullet HCl$ (459,00) | | | |
|---|---|---|---|
| Berechnet: | C, 62,80; | H, 5,93; | N, 6,10% |
| Gefunden: | C, 62,95; | H, 6,28; | N, 5,69%. |

## Beispiel 12

(±)-cis-3-(Acetyloxy)-2,3-dihydro-5-[2-(dimethylamino)äthyl]-2-(4-methoxyphenyl)naphtho[2,1-b][1,4]-thiazepin-4(5H)-on

Ein Gemisch von 1,5 g (0,0035 Mol) (±)-cis-2,3-Dihydro-3-hydroxy-5-[2-(dimethylamino)äthyl]-2-(4-methoxyphenyl)naphtho[2,1-b][1,4]thiazepin-4(5H)-on und 25 ml Essigsäureanhydrid wird während 17 Stunden auf 100° erhitzt. Das überschüssige Reagens wird unter vermindertem Druck entfernt, und der Rückstand zwischen verdünnter Ammoniumhydroxidlösung und Aethylacetat verteilt. Die Aethylacetatlösung wird mit 50 ml Wasser gewaschen und über Magnesiumsulfat getrocknet. Entfernen des Lösungsmittels liefert 1,2 g (73%) (±)-cis-3-(Acetyloxy)-2,3-dihydro-5-[2-(dimethylamino)äthyl]-2-(4-methoxyphenyl)-naphtho[2,1-b]- [1,4]thiazepin-4(5H)-on. Für die Analyse wird eine Probe dieser Verbindung aus Aether umkristallisiert, Smp. 168-169°.

| $C_{16}H_{28}N_2O_4S$ (464,58) | | | |
|---|---|---|---|
| Berechnet: | C, 67,22; | H, 6,07; | N, 6,03% |
| Gefunden: | C, 67,06; | H, 6,27; | N, 5,97%. |

## Beispiel 13

(±)-cis-3-(Acetyloxy)-2,3-dihydro-5-[2-(dimethylamino)äthyl]-2-(4-methoxyphenyl)naphtho[2.,1-b][1,4]-thiazepin-4(5H)-on-(E)-2-butendioat

1,9 g (0,004 Mol) des Endprodukts von Beispiel 12 liefert nach Umsetzen mit 0,47 g (0,004 Mol) Fumarsäure in Aceton, 2,0 g (87%) (±)-cis-3-(Acetyloxy)-2,3-dihydro-5-[2-(dimethylamino)äthyl]-2-(4-methoxyphenyl)naphtho[2,1-b][1,4]thiazepin-4(5H)-on-(E)-2-butendioat. Eine Analysenprobe wird aus Aethanol umkristallisiert, Smp. 221-222°.

| $C_{26}H_{28}N_2O_4S \bullet C_4H_4O_4$ (580,65) | | | |
|---|---|---|---|
| Berechnet: | C, 62,06; | H, 5,55; | N, 4,82% |
| Gefunden: | C, 62,26; | H, 5,71; | N, 4,85%. |

## Beispiel 14

(±)-trans-2,3-Dihydro-3-hydroxy-5-[2-(dimethylamino)äthyl-2-(4-methoxyphenyl)naphtho[2,1-b][1,4]thiazepin-4(5H)-on

Ein Gemisch von 2,4 g (0,0068 Mol) (±)-trans-2,3-Dihydro-3-hydroxy-2-(4-methoxyphenyl)naphtho[2,1-b][1,4]thiazepin-4(5H)-on, 1,0 g Kaliumcarbonat (pulverisiert) und 0,8 g (0,0068 Mol) 2-Dimethylaminoäthylchlorid in 60 ml Aethylacetat wird unter Rühren 2 Stunden zum Rückfluss erhitzt. Danach werden in jeweils 2-stündigen Intervallen dreimal zusätzlich je 0,2 g 2-Dimethylaminoäthylchlorid zugegeben. Das Reaktionsgemisch wird insgesamt während 12 Stunden zum Rückfluss erhitzt, danach auf Raumtemperatur abgekühlt und filtriert. Das Filtrat wird mit 150 ml Aethylacetat verdünnt und mit Wasser gewaschen. Die Aethylacetatlösung wird über Magnesiumsulfat getrocknet, und das Lösungsmittel entfernt, wobei man 1,9 g (66%) (±)-trans-2,3-Dihydro-3-hydroxy-5-[2-(dimethylamino)äthyl]-2-(4-methoxyphenyl)naphtho[2,1-b][1,4]thiazepin-4-(5H)-on erhält, Smp. 155-156°. Eine Analysenprobe wird aus Aceton umkristallisiert, Smp. 155-156°.

| $C_{24}H_{26}N_2O_3S$ (422,54) | | | |
|---|---|---|---|
| Berechnet: | C, 68,22; | H, 6,20; | N, 6,63% |
| Gefunden: | C, 68,44; | H, 6,28; | N, 6,59%. |

Beispiel 15

(±)-trans-2,3-Dihydro-3-hydroxy-5-[2-(dimethylamino)äthyl]-2-(4-methoxyphenyl)naphtho[2,1-b][1,4]thiazepin-4(5H)-on-hydrochlorid-semihydrat

Eine Probe des Endprodukts von Beispiel 14 liefert nach Umsetzen mit wasserfreiem Chlorwasserstoff in Aceton (±)-trans-2,3-Dihydro-3-hydroxy-5-[2-(dimethylamino)äthyl]-2-(4-methoxyphenyl)naphtho[2,1-b]-[1,4]thiazepin-4(5H) -on-hydrochlorid-semihydrat, Smp. 193-194°. Eine Analysenprobe wird aus Aceton umkristallisiert, Smp. 193-194°.

| $C_{24}H_{26}N_2O_3S \bullet HCl \bullet 0,5\ H_2O$ (468,01) | | | |
|---|---|---|---|
| Berechnet: | C, 61,59; | H, 6,03; | N, 5,99% |
| Gefunden: | C, 61,74; | H, 6,01; | N, 5,88%. |

Beispiel 16

(±)-trans-3-(Acetyloxy)-2,3-dihydro-5-[2-(dimethylamino)äthyl]-2-(4-methoxyphenyl)naphtho[2,1-b][1,4]-thiazepin-4(5H)-on

Zu einer Lösung von 1,0 g (0,0024 Mol) (±)-trans-2,3-Dihydro-3-hydroxy-5-[2-(dimethylamino)äthyl]-2-(4-methoxyphenyl)naphtho[2,1-b][1,4]thiazepin-4(5H)-on in 20 ml Pyridin wird tropfenweise 0,5 g (0,006 Mol) Acetylchlorid bei Eisbadtemperatur gegeben. Das Reaktionsgemisch wird bei Eisbadtemperatur 17 Stunden gerührt und danach zur Trockene eingedampft. Der Rückstand wird zwischen verdünnter Ammoniumhydroxidlösung und Aethylacetat verteilt. Die Aethylacetatlösung wird mit 50 ml Wasser gewaschen und über Magnesiumsulfat getrocknet. Entfernen des Lösungsmittels liefert 1,0 g (92%) (±)-trans-3-(Acetyloxy)-2,3-dihydro-5-[2-(dimethylamino)äthyl]-2-(4-methoxyphenyl)naphtho[2,1-b][1,4]thiazepin-4(5H)-on. Eine Analysenprobe wird aus Aether umkristallisiert, Smp. 170-171°.

| $C_{26}H_{28}N_2O_4S$ (464,58) | | | |
|---|---|---|---|
| Berechnet: | C, 67,22; | H, 6,07; | N, 6,03% |
| Gefunden: | C, 67,23; | H, 6,27; | N, 6,04%. |

## Beispiel 17

(±)-trans-3-(Acetyloxy)-2,3-dihydro-5-[2-(dimethylamino)äthyl]-2-(4-methoxyphenyl)naphtho[2,1-b][1,4]-thiazepin-4(5H)-on-(E)-2-butendioat-monohydrat

0,5 g (0,0001 Mol) des Endprodukts von Beispiel 16 liefert nach Umsetzen mit 0,12 g (0,0001 Mol) Fumarsäure in Aceton, 0,6 g (93%) (±)-trans-3-(Acetyloxy)-2,3-dihydro-5-[2-(dimethylamino)äthyl]-2-(4-methoxyphenyl)naphtho[2,1-b][1,4]thiazepin-4(5H)-on-(E)-2-butendioat-monohydrat, Smp. 155-156°.

| $C_{26}H_{28}N_2O_4S{\bullet}C_4H_4O_4{\bullet}H_2O$ (598,68) | | | |
|---|---|---|---|
| Berechnet: | C, 60,19; | H, 5,72; | N, 4,68% |
| Gefunden: | C, 60,09; | H, 5,55; | N, 4,40%. |

## Beispiel 18

Auftrennung von (±)-cis-2,3-Dihydro-3-hydroxy-5-[2-(dimethylamino)äthyl]-2-(4-methoxyphenyl)naphtho[2,1-b][1,4]thiazepin-4(5H)-on

Eine heisse Lösung von 17,7 g (0,042 Mol) racemischem cis-2,3-Dihydro-3-hydroxy-5-[2-(dimethylamino)äthyl]-2-(4-methoxyphenyl)naphtho[2,1-b][1,4]thiazepin-4(5H)-on in 177 ml Acetonitril wird mit einer heissen Lösung von 14,59 g (0,042 Mol) S-(+)-1,1′-Binaphthyl-2,2′-diyl-hydrogenphosphat in 730 ml Acetonitril versetzt. Man lässt die klare Lösung bei Raumtemperatur während 17 Stunden kristallisieren. Die Kristalle werden dann abfiltriert, mit Acetonitril gewaschen und getrocknet, wobei man 11,7 g (72%) (-)-cis-2,3-Dihydro-3-hydroxy-5-[2-(dimethylamino)äthyl]-2-(4-methoxyphenyl)naphtho[2,1-b][1,4]thiazepin-4-(5H)-on-(S)-4-hydroxydinaphtho[2,1-d:1′,2′-f][1,3,2]dioxaphosphepin-4-oxid erhält, Smp. 179-181°, $[\alpha]_D^{25}$ + 145,96° (c 0,48, MeOH).

| $C_{24}H_{26}N_2O_3{\bullet}C_{20}H_{13}O_4P$ (770,76) | | | |
|---|---|---|---|
| Berechnet: | C, 68,56; | H, 5,10; | N, 3,63% |
| Gefunden: | C, 68,40; | H, 5,27; | N, 3,60%. |

## Beispiel 19

(-)-cis-2,3-Dihydro-3-hydroxy-5-[2-(dimethylamino)äthyl]-2-(4-methoxyphenyl)naphtho[2,1-b][1,4]thiazepin-4-(5H)-on

11,7 g (0,015 Mol) (-)-cis-2,3-Dihydro-3-hydroxy-5-[2-(dimethylamino)äthyl]-2-(4-methoxyphenyl)-naphtho[2,1-b][1,4]thiazepin-4(5H)-on-(S)-4-hydroxydinaphtho[2,1-d:1′,2′-f][1,3,2]dioxaphosphepin-4- oxid in

200 ml Wasser wird mit konz. Ammoniumhydroxidlösung zersetzt. Die erhaltene Suspension wird mit dreimal 250 ml Aether extrahiert. Die vereinigten Aetherextrakte werden mit 75 ml Wasser gewaschen und über Magnesiumsulfat getrocknet. Entfernen des Lösungsmittels liefert 6,4 g (100%) (-)-cis-2,3-Dihydro-3-hydroxy-5-[2-(dimethylamino)äthyl]-2-(4-methoxyphenyl)naphtho[2,1-b][1,4]thiazepin-4(5H)-on. Eine Analysenprobe wird aus Aether kristallisiert, Smp. 40-41°, $[\alpha]_D^{25}$ -183,31° (c 0,91, MeOH). Ein 100 MHz NMR-Spektrum dieser Verbindung in Chloroform in Gegenwart eines chiralen Shift-Reagens [Eu(TFC)$_3$] zeigt an, dass die Substanzprobe enantiomer rein ist.

| $C_{24}H_{26}N_2O_3S$ (422,54) | | | |
|---|---|---|---|
| Berechnet: | C, 68,23; | H, 6,20; | N, 6,63% |
| Gefunden: | C, 68,36; | H, 6,26; | N, 6,59%. |

## Beispiel 20

(-)-cis-2,3-Dihydro-3-hydroxy-5-[2-(dimethylamino)äthyl]-2-(4-methoxyphenyl)naphtho[2,1-b][1,4]thiazepin-4-(5H)-on-monohydrochlorid-semihydrat

Eine Probe des Endprodukts von Beispiel 19 liefert nach Umsetzen mit wasserfreiem Chlorwasserstoff in Aethylacetat amorphes (-)-cis-2,3-Dihydro-3-hydroxy-5-[2-(dimethylamino)äthyl]-2-(4-methoxyphenyl)-naphtho[2,1-b][1,4]thiazepin-4(5H)-on-monohydrochlorid-semihydrat, $[\alpha]_D^{25}$ -170,02° (c 0,95, MeOH).

| $C_{24}H_{26}N_2O_3S \bullet HCl \bullet 0.5\ H_2O$ (468,01) | | | |
|---|---|---|---|
| Berechnet: | C, 61,59; | H, 6,03; | N, 5,99% |
| Gefunden: | C, 61,88; | H, 6,10; | N, 5,96%. |

## Beispiel 21

(+)-cis-2,3-Dihydro-3-hydroxy-5-[2-(dimethylamino)äthyl]-2-(4-methoxyphenyl)naphtho[2,1-b][1,4]thiazepin-4(5H)-on-R-(-)-BNHP

Die vereinigten Mutterlaugen, welche man bei der Herstellung des Salzes der (-)-Base mit S-(+)-1,1'-Binaphthyl-2,2'-diyl-hydrogenphosphat erhält, werden zur Trockene eingedampft. Der Rückstand wird in 200 ml Wasser mit konz. Ammoniumhydroxidlösung zersetzt, und die erhaltene Suspension mit dreimal 350 ml Aether extrahiert. Die vereinigten Extrakte werden mit 100 ml Wasser gewaschen und über Magnesiumsulfat getrocknet. Entfernen des Lösungsmittels liefert 10,60 g rohes (+)-cis-2,3-Dihydro-3-hydroxy-5-[2-(dimethylamino)äthyl]-2-(4-methoxyphenyl)naphtho[2,1-b][1,4]- thiazepin-4(5H)-on. 10,6 g (0,025 Mol) dieser Verbindung werden in 100 ml heissem Acetonitril gelöst und mit einer heissen Lösung von 8,68 g (0,025 Mol) R-(-)-1,1'- Binaphthyl-2,2'-diyl-hydrogenphosphat in 400 ml Acetonitril versetzt. Man lässt die Lösung während 17 Stunden bei Raumtemperatur kristallisieren. Die Kristalle werden dann abfiltriert, mit Acetonitril ewaschen und getrocknet, wobei man 13,4 g (83%) (+)-cis-2,3-Dihydro-3-hydroxy-5-[2-(dimethyamino)-äthyl]-2-(4-methoxyphenyl)naphtho[2,1-b][1,4]thiazepin-4(5H)-on-(R)-4-hydroxydinaphtho[2,1-d:1',2'-f][1,3,2]-dioxaphosphepin-4-oxid erhält, Smp. 277-279°, $[\alpha]_D^{25}$ -145,96° (c 1,03, MeOH).

| $C_{24}H_{26}N_2O_3S•C_{20}H_{13}O_4P$ (770,76) | | | |
|---|---|---|---|
| Berechnet:<br>Gefunden: | C, 68,56;<br>C, 68,28; | H, 5,10;<br>H, 5,06; | N, 3,63%<br>N, 3,73%. |

Beispiel 22

(+)-cis-2,3-Dihydro-3-hydroxy-5-[2-(dimethylamino)äthyl]-2-(4-methoxyphenyl)naphtho[2,1-b][1,4]thiazepin-4(5H)-on

13,5 g (0,018 Mol) (+)-cis-2,3-Dihydro-3-hydroxy-5-[2-(dimethylamino)äthyl]-2-(4-methoxyphenyl)-naphtho[2,1-b][1,4]thiazepin-4(5H)-on-(R)-4-hydroxydinaphtho[2,1-d:1′,2′-f][1,3,2]dioxaphosphepin-4-oxid in 200 ml Wasser werden mit konz. Ammoniumhydroxidlösung zersetzt. Die erhaltene Suspension wird mit dreimal 250 ml Aether extrahiert. Die vereinigten Aetherextrakte werden mit 75 ml Wasser gewaschen und über Magnesiumsulfat getrocknet. Entfernen des Lösungsmittels liefert 7,4 g (100%) (+)-cis-2,3-Dihydro-3-hydroxy-5-[2-(dimethylamino)äthyl]-2-(4-methoxyphenyl)naphtho[2,1-b][1,4]thiazepin-4(5H)-on. Eine Analysenprobe wird aus Aether/Petroläther kristallisiert, Smp. 40-41°, $[\alpha]_D^{25}$ + 185,63° (c 1,05, MeOH).

| $C_{24}H_{26}N_2O_3S$ (422,54) | | | |
|---|---|---|---|
| Berechnet:<br>Gefunden: | C, 68,23;<br>C, 67,82; | H, 6,20;<br>H, 6,15; | N, 6,63%<br>N, 6,65%. |

Beispiel 23

(+)-cis-2,3-Dihydro-3-hydroxy-5-[2-(dimethylamino)äthyl]-2-(4-methoxyphenyl)naphtho[2,1-b,[1,4]thiazepin-4(5H) -on-hydrochlorid-semihydrat

Eine Probe des Endprodukts von Beispiel 22 liefert nach Umsetzen mit wasserfreiem Chlorwasserstoff in Aethylacetat amorphes (+)-cis-2,3-Dihydro-3-hydroxy-5-[2-(dimethylamino)äthyl]-2-(4-methoxyphenyl)-naphtho[2,1-b][1,4]thiazepin-4(5H)-on-hydrochlorid-semihydrat, $[\alpha]_D^{25}$ + 175,81° (c 0,99, MeOH).

| $C_{24}H_{26}N_2O_3S•HCl•0,5\ H_2O$ | | | |
|---|---|---|---|
| Berechnet:<br>Gefunden: | C, 61,59;<br>C, 62,03; | H, 6,03;<br>H, 6,16; | N, 5,99%<br>N, 6,05%. |

Beispiel 24

(-)-cis-3-(Acetyloxy)-2,3-dihydro-5-[2-(dimethylamino)äthyl]-2-(4-methoxyphenyl)naphtho[2,1-b][1,4]thiazepin-4(5H)-on

Zu einer Lösung von 2,5 g (0,0059 Mol) (-)-cis-2,3-Dihydro-3-hydroxy-5-[2-(dimethylamino)äthyl]-2-(4-methoxyphenyl)naphtho[2,1-b][1,4]thiazepin-4(5H)-on in 25 ml Pyridin werden tropfenweise 1,2 g Acetylchlorid bei Eisbadtemperatur gegeben. Das Gemisch wird 17 Stunden bei Eisbadtemperatur gerührt und

danach zur Trockene eingedampft. Der Rückstand wird zwischen verdünnter Ammoniumhydroxidlösung und Aethylacetat verteilt. Die Aethylacetatlösung wird mit Wasser gewaschen und über Magnesiumsulfat getrocknet. Entfernen des Lösungsmittels liefert 2,7 g Produkt, welches nach Umkristallisation aus Aether 2,5 g (91%) (-)-cis-3-(Acetyloxy)-2,3-dihydro-5-[2-(dimethylamino)äthyl]-2-(4-methoxyphenyl)naphtho-[2,1-b]-[1,4]thiazepin-4(5H)-on liefert, Smp. 124-125°, $[\alpha]_D^{25}$ -226,37° (c 1,00, MeOH).

| $C_{26}H_{28}N_2O_4S$ (464,56) | | | |
|---|---|---|---|
| Berechnet: | C, 67,22; | H, 6,08; | N, 6,03% |
| Gefunden: | C, 67,09; | H, 6,13; | N, 6,03%. |

Beispiel 25

(-)-cis-3-(Acetyloxy)-2,3-dihydro-5-[2-(dimethylamino)äthyl]-2-(4-methoxyphenyl)naphtho[2,1-b][1,4]thiazepin-4(5H)-on-(E)-2-butendioat

2,2 g (0,0047 Mol) des Endprodukts von Beispiel 24 liefert nach Umsetzen mit 0,55 g (0,0047 Mol) Fumarsäure in Aceton 2,1 g (76%) (-)-cis-3-(Acetyloxy)-2,3-dihydro-5-[2-(dimethylamino)äthyl]-2-(4-methoxyphenyl)naphtho[2,1-b][1,4]thiazepin-4(5H)-on-(E)-2-butendioat, Smp. 204-205°, $[\alpha]_D^{25}$ - 189,82° (c 1,12, MeOH).

| $C_{26}H_{28}N_2O_4S.C_4H_4O_4$ (580,63) | | | |
|---|---|---|---|
| Berechnet: | C, 62,06; | H, 5,56; | N, 4,83% |
| Gefunden: | C, 62,18; | H, 5,58; | N, 4,84%. |

Beispiel 26

(+)-cis-3-(Acetyloxy)-2,3-dihydro-5-[2-(dimethylamino)äthyl]2-(4-methoxyphenyl)naphtho[2,1-b][1,4]-thiazepin-4(5H)-on

Zu einer Lösung von 3,6 g (0,0085 Mol) (+)-cis-2,3-Dihydro-3-hydroxy-5-[2-(dimethylamino)äthyl]-2-(4-methoxyphenyl)naphtho[2,1-b][1,4]thiazepin-4(5H)-on in 36 ml Pyridin werden tropfenweise 2,0 g Acetylchlorid bei Eisbadtemperatur gegeben. Das Reaktionsgemisch wird danach bei Eisbadtemperatur 17 Stunden gerührt und dann zur Trockene eingedampft. Der Rückstand wird zwischen verdünnter Ammoniumhydroxidlösung und Aethylacetat verteilt. Die Aethylacetatlösung wird mit Wasser gewaschen und über Magnesiumsulfat getrocknet. Entfernen des Lösungsmittels liefert 3,9 g (98%) (+)-cis-3-(Acetyloxy)-2,3-dihydro-5-[2-(dimethylamino)äthyl]-2-(4-methoxyphenyl)naphtho[2,1-b][1,4]thiazepin-4(5H)-on. Eine Analysenprobe wird aus Aether umkristallisiert, Smp. 123-124°, $[\alpha]_D^{25}$ + 222,49° (c 1,02, MeOH).

| $C_{26}H_{28}N_2O_4S$ (464,56) | | | |
|---|---|---|---|
| Berechnet: | C, 67,22; | H, 6,08; | N, 6,03% |
| Gefunden: | C, 67,13; | H, 6,17; | N, 5,89%. |

Beispiel 27

(+)-cis-3-(Acetyloxy)-2,3-dihydro-5-[2-(dimethylamino)äthyl]-2-(4-methoxyphenyl)naphtho[2,1-b][1,4]-thiazepin-4(5H)-on-(E)-2-butendioat

3,8 g (0,0082 Mol) des Endprodukts von Beispiel 26 liefert nach Umsetzen mit 0,95 g (0,0081 Mol) Fumarsäure in Aceton 4,0 g (84%) (+)-cis-3-(Acetyloxy)-2,3-dihydro-5-[2-(dimethylamino)äthyl]-2-(4-methoxyphenyl)naphtho[2,1-b][1,4]thiazepin-4(5H)-on-(E)-2-butendioat. Eine Analysenprobe wird aus Aceton umkristallisiert, Smp. 203-204°, $[\alpha]_D^{25}$ + 190,58° (c 0,98, MeOH).

| $C_{26}H_{28}N_2O_4S \bullet C_4H_4O_4$ (580,63) | | | |
|---|---|---|---|
| Berechnet: | C, 62,06; | H, 5,56; | N, 4,83% |
| Gefunden: | C, 62,07; | H, 5,49; | N, 4,88%. |

Beispiel 28

(±)-cis-3-[(Aethoxycarbonyl)oxy]-2,3-dihydro-2-(4-methoxyphenyl)-5-[2-(dimethylamino)äthyl]naphtho[2,1-b]-[1,4]thiazepin-4(5H)-on

Zu einer in einem Eisbad gekühlten Lösung von 2,0 g (0,0047 Mol) (±)-cis-2,3-Dihydro-3-hydroxy-5-[2-(dimethylamino)äthyl]-2-(4-methoxyphenyl)naphtho[2,1-b][1,4]thiazepin-4(5H)-on in 25 ml über Kaliumhydroxid getrocknetem Pyridin wird tropfenweise 0,65 g (0,006 Mol) Aethylchloroformat gegeben, und das Reaktionsgemisch über Nacht im Kühlschrank stehen gelassen. Das Reaktionsgemisch wird danach unter vermindertem Druck eingedampft, und der Rückstand zwischen Aethylacetat und verdünnter Ammoniumhydroxidlösung verteilt. Die Aethylacetatlösung wird mit gesättigter Kochsalzlösung gewaschen und über Magnesiumsulfat getrocknet. Entfernen des Lösungsmittels liefert 2,3 g (98%) (±)-cis-3-[(Aethoxycarbonyl)-oxy]-2,3-dihydro-2-(4-methoxyphenyl)-5-[2-(dimethylamino)äthyl]naphtho[2,1-b][1,4]thiazepin-4(5H)-on. Eine Analysenprobe wird aus Aether umkristallisiert, Smp. 125-126°.

| $C_{27}H_{30}N_2O_5S$ (494,53) | | | |
|---|---|---|---|
| Berechnet: | C, 65,57; | H, 6,11; | N, 5,67% |
| Gefunden: | C, 65,50; | H, 6,19; | N, 5,69%. |

Beispiel 29

(±)-cis-3-[(Aethoxycarbonyl)oxy]-2,3-dihydro-2-(4-methoxyphenyl)-5-[2-(dimethylamino)äthyl]naphtho[2,1-b]-[1,4]thiazepin-4(5H)-on-(E)-2-butendioat

0,5 g (0,001 Mol) des Endprodukts von Beispiel 28 liefert nach Umsetzen mit 0,120 g (0,001 Mol) Fumarsäure in Aceton, 0,5 g (82%) (±)-cis-3-[(Aethoxycarbonyl)oxy]-2,3-dihydro-2-(4-methoxyphenyl)-5-[2-(dimethylamino)äthyl]naphtho[2,1-b][1,4]thiazepin-4(5H)-on-(E)-2-butendioat. Eine Analysenprobe wird aus Aceton umkristallisiert, Smp. 205-206°.

| $C_{27}H_{30}N_2O_5S \bullet C_4H_4O_4$ (610,66) | | | |
|---|---|---|---|
| Berechnet: | C, 60,97; | H, 5,61; | N, 4,59% |
| Gefunden: | C, 60,96; | H, 5,55; | N, 4,50%. |

Beispiel 30

(±)-cis-2,3-Dihydro-3-methoxy-5-[2-(dimethylamino)äthyl]-2-(4-methoxyphenyl)naphtho[2,1-b][1,4]thiazepin-4-(5H)-on

Zu einem Gemisch von 3,2 g (0,008 Mol) (±)-cis-2,3-Dihydro-3-hydroxy-5-[2-(dimethylamino)äthyl]-2-(4-methoxyphenyl)naphtho[2,1-b][1,4]thiazepin-4(5H)-on und 60 ml Benzol wird 0,5 g Natriumhydrid (50%-ig in Mineralöl) gegeben, und das Reaktionsgemisch 1 Stunde zum Rückfluss erhitzt. Nach Abkühlen auf Raumtemperatur gibt man 1,3 g (0,01 Mol) Dimethylsulfat tropfenweise zu und rührt das Reaktiongemisch 17 Stunden bei dieser Temperatur. Danach wird das Reaktionsgemisch zwischen verdünnter Ammoniumhydroxidlösung und Aethylacetat verteilt. Die organische Phase wird mit gesättigter Kochsalzlösung gewaschen und über Magnesiumsulfat getrocknet. Entfernen des Lösungsmittels liefert 2,3 g Produkt, welches nach Umkristallisation aus Aethylacetat 1,9 g (58%) (±)-cis-2,3-Dihydro-3-methoxy-5-[2-(dimethylamino)-äthyl]-2-(4-methoxyphenyl)naphtho-[2,1-b][1,4]thiazepin-4(5H)-on liefert, Smp. 177-178°.

| $C_{25}H_{28}N_2O_3S$ (436,49) | | | |
|---|---|---|---|
| Berechnet: | C, 68,79; | H, 6,47; | N, 6,42% |
| Gefunden: | C, 69,07; | H, 6,49; | N, 6,16%. |

Beispiel 31

(±)-cis-2,3-Dihydro-3-methoxy-5-[2-(dimethylamino)äthyl]-2-(4-methoxyphenyl)naphtho[2,1-b][1,4]thiazepin-4-(5H)-on-(E)-2-butendioat

0,3 g (0,0007 Mol) des Endprodukts von Beispiel 30 liefert nach Umsetzen mit 0,08 g (0,0007 Mol) Fumarsäure in Aceton, 0,3 g (79%) (±)-cis-2,3-Dihydro-3-methoxy-5-[2-(dimethylamino)äthyl]-2-(4-methoxyphenyl)naphtho[2,1-b][1,4]thiazepin-4(5H)-on-(E)-2-butendioat. Eine Analysenprobe wird aus Aceton umkristallisiert, Smp. 242-243°.

| $C_{25}H_{28}N_2O_3S \bullet C_4H_4O_4$ (552,62) | | | |
|---|---|---|---|
| Berechnet: | C, 63,03; | H, 5,84; | N, 5,07% |
| Gefunden: | C, 63,01; | H, 5,69; | N, 5,06%. |

Beispiel 32

(±)-cis-2,3-Dihydro-3-(2-methoxy-1-oxoäthoxy)-2-(4-methoxyphenyl)-5-[2-(dimethylamino)äthyl]naphtho[2,1-b][1,4]thiazepin-4(5H)-on

Zu einer Lösung von 2,5 g (0.0059 Mol) (±)-cis-2,3-Dihydro-3-hydroxy-5-[2-(dimethylamino)äthyl]-2-(4-methoxyphenyl)naphtho[2,1-b][1,4]thiazepin-4(5H)-on in 25 ml über Kaliumhydroxid getrocknetem Pyridin werden tropfenweise bei Eisbadtemperatur 1,2 g (0,011 Mol) Methoxyacetylchlorid gegeben, und das Reaktionsgemisch über Nacht bei dieser Temperatur gerührt. Danach wird das Gemisch unter vermindertem Druck eingedampft, und der Rückstand zwischen Aethylacetat und verdünnter Ammoniumhydroxidlösung verteilt. Die Aethylacetatlösung wird mit 50 ml gesättigter Kochsalzlösung gewaschen und über Magnesiumsulft getrocknet. Entfernen des Lösungsmittels ergibt 2,7 g Produkt, welches nach Umkristallisation aus Aether 2,2 g (76%) (±)-cis-2,3-Dihydro-3-(2-methoxy-1-oxoäthoxy)-2-(4-methoxyphenyl)-5-[2-(dimethylamino)äthyl]naphtho[2,1-b][1,4]thiazepin-4(5H)-on liefert, Smp. 131-132°.

| $C_{27}H_{30}N_2O_5S$ (494,60) | | | |
|---|---|---|---|
| Berechnet: | C, 65,57; | H, 6,11; | N, 5,66% |
| Gefunden : | C, 65,36; | H, 6,29; | N, 5,44%. |

## Beispiel 33

(±)-cis-2,3-Dihydro-3-(2-methoxy-1-oxoäthoxy)-2-(4-methoxyphenyl)-5-[2-(dimethylamino)äthyl]naphtho[2,1-b][1,4]thiazepin-4(5H)on-(E)-2-butendioat

1,7 g (0,0034 Mol) des Endprodukts von Beispiel 32 liefert nach Umsetzen mit 0,4 g (0,0034 Mol) Fumarsäure in Aceton 1,6 g (77%) (±)-cis-2,3-Dihydro-3-(2-methoxy-1-oxoäthoxy)-2-(4-methoxyphenyl)-5-[2-(dimethylamino)äthyl]naphtho[2,1-b][1,4]thiazepin-4(5H)on-(E)-2-butendioat. Eine Analysenprobe wird aus Aceton umkristallisiert, Smp. 217-218°.

| $C_{27}H_{30}N_2O_5S \bullet C_4H_4O_4$ (610,68) | | | |
|---|---|---|---|
| Berechnet: | C, 60,97; | H, 5,61; | N, 4,59% |
| Gefunden : | C, 60,96; | H, 5,84; | N, 4,62%. |

## Beispiel 34

(±)-cis-3-[(Cyclopropylcarbonyl)oxy]-2,3-dihydro-5-[2-(dimethylamino)äthyl]-2-(4-methoxyphenyl)naphtho[2,1-b][1,4]thiazepin-4(5H)-on

Zu einer Lösung von 2,5 g (0,0059 Mol) (±)-cis-2,3-Dihydro-3-hydroxy-5-[2-(dimethylamino)äthyl]-2-(4-methoxyphenyl)naphtho[2,1-b][1,4]thiazepin-4(5H)-on in 25 ml Pyridin werden tropfenweise 1,2 g (0,011 Mol) Cyclopropancarbonsäurechlorid bei Eisbadtemperatur gegeben. Die Reaktionslösung wird danach 17 Stunden im Kühlschrank stehen gelassen und unter vermindertem Druck eingedampft. Der Rückstand wird zwischen verdünnter Ammoniumhydroxidlösung und Aethylacetat verteilt. Die organische Lösung wird mit gesättigter Kochsalzlösung gewaschen und über Magnesiumsulfat getrocknet. Entfernen des Lösungsmittels ergibt 3,0 g Produkt, welches nach Umkristallisation aus Aether 2,4 g (83%) (±)-cis-3-[-(Cyclopropylcarbonyl)oxy]-2,3-dihydro-5-[2-(dimethylamino)äthyl]-2-(4-methoxyphenyl)naphtho[2,1-b][1,4]-thiazepin-4(5H)-on liefert, Smp. 160-161°.

| $C_{28}H_{30}N_2O_4S$ (490,54) | | | |
|---|---|---|---|
| Berechnet: | C, 68,55; | H, 6,16; | N, 5,71% |
| Gefunden : | C, 68,44; | H, 6,08; | N, 5,59%. |

## Beispiel 35

(±)-cis-3-[(Cyclopropylcarbonyl)oxy]-2,3-dihydro-5-[2-(dimethylamino)äthyl]-2-(4-methoxyphenyl)naphtho[2,1-b][1,4,]thiazepin-4(5H)-on-(E)-2-butendioat

2,4 g (0,005 Mol) des Endprodukts von Beispiel 34, liefert nach Umsetzen mit 0,6 g (0,0049 Mol) Fumarsäure in Aceton 2,5 g (84%) (±)-cis-3-[(Cyclopropylcarbonyl)oxy]-2,3-dihydro -5-[2-(dimethylamino)-äthyl]-2-(4-methoxyphenyl)naphtho[2,1-b][1,4]thiazepin-4(5H)-on-(E)-2-butendioat. Eine Analysenprobe wird aus Methanol umkristallisiert, Smp. 242-243°.

| $C_{28}H_{30}N_2O_4S{\bullet}C_4H_4O_4$ (606,67) | | | |
|---|---|---|---|
| Berechnet: | C, 63,36; | H, 5,65; | N, 4,62% |
| Gefunden : | C, 63,45; | H, 5,64; | N, 4,55%. |

## Beispiel 36

cis-rac-5-[2-(Dimethylamino)propyl]-2,3-dihydro-3-hydroxy-2-(4-methoxyphenyl)naphtho[2,1-b][1,4]thiazepin-4(5H)-on

Eine Mischung von 4,4 g (0,013 Mol) (±)-cis-2,3-Dihydro-3-hydroxy-2-(4-methoxyphenyl)naphtho[2,1-b]-[1,4]thiazepin-4(5H)-on, 2,0 g pulverisiertem Kaliumcarbonat und 1,7 g 3-Dimethylaminopropylchlorid in 100 ml Aethylacetat wird 2 Stunden unter Rühren zum Rückfluss erhitzt. Dann werden noch dreimal in 2-stündigen Intervallen je 0,9 g 3-Dimethylaminopropylchlorid zugegeben. Das Reaktionsgemisch wird insgesamt 12 Stunden zum Rückfluss erhitzt, dann auf Raumtemperatur abgekühlt und mit 100 ml Wasser verdünnt. Die organische Phase wird abgetrennt, mit gesättigter Kochsalzlösung gewaschen und über Magnesiumsulfat getrocknet. Entfernen des Lösungsmittels ergibt ein Produkt, welches nach Kristallisation aus Aether 2,3 g (43%) cis-rac-5-[2-(Dimethylamino)propyl]-2,3-dihydro-3-hydroxy-2-(4-methoxyphenyl)-naphtho[2,1-b][1,4]thiazepin-4(5H)-on liefert, Smp. 136-137°.

| $C_{25}H_{28}N_2O_3S$ (436,39) | | | |
|---|---|---|---|
| Berechnet: | C, 68,79; | H, 6,47; | N, 6,42% |
| Gefunden : | C, 68,60; | H, 6,43; | N, 6,18%. |

## Beispiel 37

cis-rac-5-[3-(Dimethylamino)propyl]-2,3-dihydro-3-hydroxy-2-(4-methoxyphenyl)naphtho[2,1-b][1,4]thiazepin-4(5H)-on-(E)-2-butendioat

1,0 g (0,0023 Mol) des Endprodukts von Beispiel 36 liefert nach Umsetzen mit 0,3 g (0,0025 Mol) Fumarsäure in Aceton 1,1 g (87%) cis-rac-5-[3-(Dimethylamino)propyl]-2,3-dihydro-3-hydroxy-2-(4-methoxyphenyl)naphtho[2,1-b][1,4]thiazepin-4(5H)-on (E)-2-butendioat, Smp. 227-228°.

| $C_{25}H_{28}N_2O_3S{\bullet}C_4H_4O_4$ (552,56) | | | |
|---|---|---|---|
| Berechnet: | C, 63,03; | H, 5,84; | N, 5,07% |
| Gefunden : | C, 63,04; | H, 5,96; | N, 4,89%. |

Beispiel 38

cis-rac-3-(Acetyloxy)-2,3-dihydro-2-(4-methoxyphenyl)-5-[3-(dimethylamino)propyl]naphtho[2,1-b][1,4]-thiazepin-4(5H)-on

Zu einer Lösung von 1,4 g (0,0032 Mol) (±)-cis-5-[3-(Dimethylamino)propyl]-2,3-dihydro-3-hydroxy-2-(4-methoxyphenyl)naphtho[2,1-b][1,4]thiazepin-4(5H)-on in 25 ml über Kaliumhydroxid getrocknetem Pyridin wird tropfenweise bei Eisbadtemperatur 1 g Acetylchlorid gegeben, und das Gemisch über Nacht bei dieser Temperatur gehalten. Danach wird das Reaktionsgemisch unter vermindertem Druck eingedampft, und der Rückstand zwischen Aethylacetat und verdünnter Ammoniumhydroxidlösung verteilt. Die vereinigten Aethylacetatlösungen werden mit gesättigter Kochsalzlösung gewaschen und über Magnesiumsulfat getrocknet. Entfernen des Lösungsmittels ergibt ein Produkt, welches nach Kristallisation aus Aethylacetat 1,3 g (87%) cis-rac-3-(Acetyloxy)-2,3-dihydro-2-(4-methoxyphenyl)-5-[3-(dimethylamino)propyl]naphtho[2,1-b][1,4]-thiazepin -4(5H)-on liefert, Smp. 154-155°.

| $C_{27}H_{30}N_2O_4S$ (478,59) | | | |
|---|---|---|---|
| Berechnet: | C, 67,76; | H, 6,32; | N, 5,85% |
| Gefunden : | C, 67,53; | H, 6,55; | N, 5,83%. |

Beispiel 39

cis-rac-3-(Acetyloxy)-2,3-dihydro-2-(4-methoxyphenyl)-5-[3-(dimethylamino)propyl]naphtho[2,1-b][1,4]-thiazepin-4(5H)-on-(E)-2-butendioat

Zu 1,3 g (0,0027 Mol) des Endprodukts von Beispiel 38 in 25 ml Aceton werden 0,3 g (0,0026 Mol) Fumarsäure gegeben, und die erhaltenen Kristalle abfiltriert, wobei man 1,3 g (81%) cis-rac-3-(Acetyloxy)-2,3-dihydro-2-(4-methoxyphenyl)-5-[3-(dimethylamino)propyl]naphtho[2,1-b][1,4]thiazepin-4(5H)-on (E)-2-butendioat erhält, Smp. 132-134°.

| $C_{27}H_{30}N_2O_4S \bullet C_4H_4O_4$ (594,66) | | | |
|---|---|---|---|
| Berechnet: | C, 62,62; | H, 5,76; | N, 4,71% |
| Gefunden : | C, 62,40; | H, 5,98; | N, 4,67%. |

Beispiel 40

cis-rac-5-[2-(Diäthylamino)äthyl]-2,3-dihydro-3-hydroxy-2-(4-methoxyphenyl)naphtho[2,1-b][1,4]thiazepin-4-(5H)-on-(E)-butendioat

Ein Gemisch von 4,4 g (0,013 Mol) cis-rac-2,3-Dihydro-3-hydroxy-2-(4-methoxyphenyl)naphtho[2,1-b]-[1,4]thiazepin-4(5H)-on, 2,0 g pulverisiertem Kaliumcarbonat und 1,8 g Diäthylaminoäthylchlorid in 100 ml Aethylacetat wird unter Rühren während 2 Stunden zum Rückfluss erhitzt. Dann werden noch dreimal in 2-stündigen Intervallen je 0,5 g 2-Diäthylaminoäthylchlorid zugegeben. Das Reaktionsgemisch wird insgesamt während 12 Stunden zum Rückfluss erhitzt, dann auf Raumtemperatur abgekühlt und mit 100 ml Wasser verdünnt. Die organische Phase wird abgetrennt, mit gesättigter Kochsalzlösung gewaschen und über Magnesiumsulfat getrocknet. Entfernen des Lösungsmittels ergibt 5,4 g Produkt, welches in Aceton mit 1,3

g Fumarsäure umgesetzt wird. Die erhaltenen Kristalle werden abfiltriert und getrocknet, wobei man 3,7 g (52%) rac-cis-5-[2-(Diäthylamino)äthyl]-2,3-dihydro-3-hydroxy-2-(4-methoxyphenyl)naphtho[2,1-b][1,4]-thiazepin-4(5H)-on-(E)-butendioat erhält, Smp. 195-196°.

| $C_{26}H_{30}N_2O_3S{\bullet}C_4H_4O_4$ (566,65) | | | |
|---|---|---|---|
| Berechnet: | C, 63,59; | H, 6,05; | N, 4,94% |
| Gefunden : | C, 63,53; | H, 6,08; | N, 5,04%. |

Beispiel 41

cis-rac-5-[2-(Diäthylamino)äthyl]-2,3-dihydro-3-hydroxy-2-(4-methoxyphenyl)naphtho[2,1-b][1,4]thiazepin-4-(5H)-on

Man versetzt eine Probe des (E)-2-Butendioatsalzes von Beispiel 40 in Wasser mit konzentrierter Ammoniumhydroxidlösung und extrahiert die wässrige Suspension mit Aethylacetat. Die Aethylacetatextrakte werden mit gesättigter Kochsalzlösung gewaschen und über Magnesiumsulfat getrocknet. Entfernen des Lösungsmittels ergibt die Base, welche nach Kristallisation aus Aether cis-rac-5-[2-(Diäthylamino)äthyl]-2,3-dihydro-3-hydroxy-2-(4-methoxyphenyl)naphtho[2,1-b][1,4]thiazepin-4(5H)-on liefert, Smp. 111-112°.

| $C_{26}H_{30}N_2O_3S$ (450,51) | | | |
|---|---|---|---|
| Berechnet: | C, 69,31; | H, 6,71; | N, 6,22% |
| Gefunden : | C, 69,04; | H, 6,76; | N, 6,27%. |

Beispiel 42

cis-rac-3-(Acetyloxy)-5-[2-(diäthylamino)äthyl]-2,3-dihydro-2-(4-methoxyphenyl)naphtho[2,1-b][1,4]thiazepin-4(5H)-on-(E)-2-butendioat

Eine Lösung von 1,9 g (0,0042 Mol) cis-rac-5-[2-(Diäthylamino)äthyl]-2,3-dihydro-3-hydroxy-2-(4-methoxyphenyl)naphtho[2,1-b][1,4]thiazepin,4(5H)-on in 25 ml Aceton wird mit wasserfreiem Chlorwasserstoff angesäuert und danach unter vermindertem Druck eingedampft. Der Rückstand wird in 25 ml Essigsäureanhydrid gelöst und während 17 Stunden unter Rühren auf 100° erhitzt. Das überschüssige Reagens wird unter vermindertem Druck entfernt, und der Rückstand in Wasser gelöst. Die wässrige Suspension wird mit konzentrierter Ammoniumhydroxidlösung basisch gestellt und mit dreimal 75 ml Aethylacetat extrahiert. Die vereinigten Aethylacetatextrakte werden mit 50 ml gesättigter Kochsalzlösung gewaschen und über Magnesiumsulfat getrocknet. Entfernen des Lösungsmittels ergibt 1,8 g Produkt, welches in Aceton mit 0,4 g Fumarsäure umgesetzt wird. Die Kristalle werden abfiltriert und getrocknet, wobei man 1,2 g (46%) cis-rac-3-(Acetyloxy)-5-[2-(diäthylamino)äthyl]-2,3-dihydro-2-(4-methoxyphenyl)naphtho[2,1-b][1,4]thiazepin-4(5H)-on-(E)-2-butendioat erhält, Smp. 180-181°.

| $C_{26}H_{32}N_2O_4S{\bullet}C_4H_4O_4$ (608,62) | | | |
|---|---|---|---|
| Berechnet: | C, 63,15; | H, 5,96; | N, 4,61% |
| Gefunden : | C, 63,24; | H, 6,20; | N, 4,75%. |

## Beispiel 43

cis-rac-3-(Acetyloxy)-5-[2-(diäthylamino)äthyl]-2,3-dihydro-2-(4-methoxyphenyl)naphtho[2,1-b][1,4]thiazepin-4(5H)-on

Man versetzt eine Probe des (E)-2-Butendiotsalzes von Beispiel 42 in Wasser mit konzentrierter Ammoniumhydroxidlösung und extrahiert die wässrige Suspension mit Aethylacetat. Die Aethylacetatextrakte werden mit gesätigter Kochsalzlösung gewaschen und über Magnesiumsulfat getrocknet. Entfernen des Lösungsmittels ergibt die Base von Beispiel 42, welche nach Kristallisation aus Aether cis-rac-3-(Acetyloxy)-5-[2-(diäthylamino)äthyl]-2,3-dihydro-2-(4-methoxyphenyl)naphtho[2,1-b][1,4]thiazepin-4(5H)-on liefert, Smp. 112-113°.

| $C_{28}H_{32}N_2O_4S$ (492,61) | | | |
|---|---|---|---|
| Berechnet: | C, 68,27; | H, 6,55; | N, 5,69% |
| Gefunden : | C, 68,59; | H, 6,61; | N, 5,70%. |

## Beispiel 44

### Tabletten

| Bestandteile | mg/Tablette | |
|---|---|---|
| | 100 mg | 200 mg |
| cis-(+)-3-(Acetyloxy)-2,3-dihydro-5-[2-(dimethylamino)äthyl]-2-(4-methoxyphenyl)naphtho[2,1-b][1,4]thiazepin-4(5H)-on (E)-2-butendioat | 100 | 200 |
| Lactose | 100 | 200 |
| Polyvinylpyrrolidon | 10 | 20 |
| Modifizierte Stärke | 10 | 20 |
| Magnesiumstearat | 3 | 6 |
| | 223 mg | 446 mg |

Verfahren

Man mischt den Wirkstoff, die Lactose und die modifizierte Stärke und granuliert das Gemisch mit Polyvinylpyrrolidon in Wasser oder Alkohol. Danach wird das Granulat bei 45° getrocknet. Man mahlt das getrocknete Granulat mit einer geeigneten Mühle und fügt das Magnesiumstearat zu. Das Gemisch wird dann 3 Minuten vermischt und auf einer geeigneten Presse zu Tabletten verpresst.

Beispiel 45

Kapseln

| Bestandteile | mg/Kapsel | |
|---|---|---|
| | 100 mg | 200 mg |
| cis-( + )-3-(Acetyloxy)-2,3-dihydro-5-[2-(dimethylamino)äthyl]-2-(4-methoxyphenyl)naphtho[1,2-b][1,4]thiazepin-4(5H)-on (E)-2-butendioat | 100 | 200 |
| Vorgelatinisierte Maisstärke | 50 | 80 |
| Modifizierte Stärke | 10 | 20 |
| Talk | 20 | 20 |
| Magnesiumstearat | 1 | 1 |
| | 181 mg | 321 mg |

Verfahren

Man mischt den Wirkstoff mit der vorgelatinisierten Maisstärke und der modifizierten Stärke und granuliert feucht mit Wasser. Das Granulat wird über Nacht bei 45° getrocknet und mit einer geeigneten Mühle vermahlen. Danach wird der Talk und das Magnesiumstearat zugegeben, das Gemisch 5 Minuten vermischt und in geeignete Kapseln abgefüllt.

## Beispiel 46

### Parenterale Lösung

| Bestandteile | mg/ml |
|---|---|
| cis-rac-2,3-Dihydro-3-hydroxy-5-[2-(dimethylamino)äthyl]-2-(4-methoxyphenyl)naphtho[2,1-b][1,4]thiazepin-4(5H)-on-hydrochlorid | 10 |
| Benzylalkohol | 10 |
| Sorbitol | 38 |
| Natriumhydroxid und/oder Salzsäure zum Justieren des pH (3-7) | q.s. |
| Wasser (für Injektion) | q.s. ad 1,0 ml |
| Gesamtvolumen | 1,0 ml |

In der obigen parenteralen Lösung bedeutet "q.s." eine genügende Menge.

## Ansprüche

1. Naphtho[2,1-b][1,4]thiazepin-4(5H)-one der allgemeinen Formel

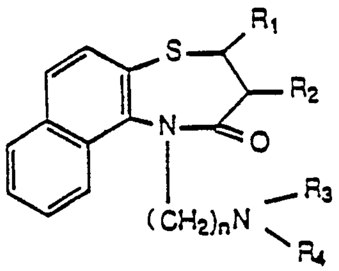

I

worin $R_1$ Phenyl mit 1-3 Niederalkoxy- oder Halogensubstituenten, $R_2$ Hydroxy, Niederalkoxy, Niederalkanoyloxy, Niedercycloalkylcarbonyloxy, -O-COO-($C_1$-$C_5$-Alkyl) oder -O-CO($CH_2$)$_m$-O-($C_1$-$C_3$-Alkyl), $R_3$ und $R_4$ unabhängig voneinander je Niederalkyl oder Phenylniederalkyl oder zusammen einen Piperidin- oder Pyrrolidinring, n 2-4 und m 1 oder 2 bedeuten,
und pharmazeutisch verwendbare Säureadditionssalze davon.

2. Verbindungen gemäss Anspruch 1, welche die cis-Konfiguration aufweisen.

3. Verbindungen gemäss Anspruch 2, welche die (+)-cis-Konfiguration aufweisen.

4. Verbindungen gemäss einem der Ansprüche 1-3, worin $R_1$ 4-Niederalkoxyphenyl und $R_2$ Hydroxy, Niederalkoxy, Niedercycloalkylcarbonyloxy, -O-CO($CH_2$)$_m$-O-($C_1$-$C_3$-Alkyl) oder -OCOO-($C_1$-$C_5$-Alkyl) bedeuten.

5. Verbindungen gemäss einem der Ansprüche 1-3, worin $R_1$ 4-Niederalkoxphenyl, $R_2$ Niederalkanoyloxy, n 2 oder 3 und $R_3$ und $R_4$ unabhängig voneinander je Niederalkyl bedeuten.

6. Verbindungen gemäss Anspruch 5, worin $R_1$ 4-Methoxyphenyl oder 4-Aethoxyphenyl, $R_2$ Hydroxy, Acetyloxy oder Propionyloxy, n 2 und $R_3$ und $R_4$ je Methyl oder Aethyl bedeuten.

7. Verbindungen gemäss Anspruch 6, worin $R_1$ 4-Methoxyphenyl, $R_2$ Hydroxy oder Acetyloxy und $R_3$ und $R_4$ je Methyl bedeuten.

8. cis-rac-3-(Acetyloxy)-2,3-dihydro-5-[2-(dimethylamino)äthyl]-2-(4-methoxyphenyl)naphtho[2,1-b][1,4]-thiazepin-4(5H)-on.

9. cis-rac-2,3-Dihydro-3-hydroxy-5-[2-(dimethylamino)äthyl]-2-(4-methoxyphenyl)naphtho[2,1-b][1,4]-thiazepin-4(5H)-on.

10. cis-(+)-3-(Acetyloxy)-2,3-dihydro-5-[2-(dimethylamino)äthyl]-2-(4-methoxyphenyl)naphtho[2,1-b]-[1,4]thiazepin-4(5H)-on.

11. cis-(+)-2,3-Dihydro-3-hydroxy-5-[2-(dimethylamino)äthyl]-2-(4-methoxyphenyl)naphtho[2,1-b][1,4]-thiazepin-4(5H)-on.

12. trans-rac-2,3-Dihydro-3-hydroxy-5-[2-(dimethylamino)äthyl]-2-(4-methoxyphenyl)naphtho[2,1-b][1,4]-thiazepin-4(5H)-on,
trans-rac-3-(Acetyloxy)-2,3-dihydro-5-[2-(dimethylamino)äthyl]-2-(4-methoxyphenyl)naphtho[2,1-b][1,4]-thiazepin-4(5H)-on,
cis-(-)-2,3-Dihydro-3-hydroxy-5-[2-(dimethylamino)äthyl]- 2-(4-methoxyphenyl)naphtho[2,1-b][1,4]thiazepin-4-(5H)-on,
cis-(+)-2,3-Dihydro-3-hydroxy-5-[2-(dimethylamino)äthyl]-2-(4-methoxyphenyl)naphtho[2,1-b][1,4]thiazepin-4(5H)-on,
cis-(-)-3-(Acetyloxy)-2,3-dihydro-5-[2-(dimethylamino)äthyl]-2-(4-methoxyphenyl)naphtho[2,1-b][1,4]thiazepin-4(5H)-on,
cis-(±)-3-[(Aethoxycarbonyl)oxy]-2,3-dihydro-2-(4-methoxyphenyl)-5-[2-(dimethylamino)äthyl]naphtho[2,1-b]-[1,4]thiazepin-4(5H)-on,
cis-(±)-2,3-Dihydro-3-methoxy-5-[2-(dimethylamino)äthyl]-2-(4-methoxyphenyl)naphtho[2,1-b][1,4]thiazepin-4-(5H)-on,
cis-(±)-2,3-Dihydro-3-(2-methoxy-1-oxoäthoxy)-2-(4-methoxyphenyl)-5-[2-(dimethylamino)äthyl]naphtho[2,1-b][1,4]thiazepin-4(5H)-on,
cis-(±)-3-[(Cyclopropylcarbonyl)oxy]-2,3-dihydro-5-[2-(dimethylamino)äthyl]-2-(4-methoxyphenyl)naphtho[2,1-b][1,4]thiazepin-4(5H)-on,
cis-rac-5-[2-(Dimethylamino)propyl]-2,3-dihydro-3-hydroxy-2-(4-methoxyphenyl)naphtho[2,1-b][1,4]thiazepin-4(5H)-on,
cis-rac-3-(Acetyloxy)-2,3-dihydro-2-(4-methoxyphenyl)-5-[3-(dimethylamino)propyl]naphtho[2,1-b][1,4]-thiazepin-(5H)-on,
cis-rac-5-[2-(Diäthylamino)äthyl]-2,3-dihydro-3-hydroxy-2-(4-methoxyphenyl)naphtho[2,1-b][1,4]thiazepin-4-(5H)-on oder
cis-rac-3-(Acetyloxy)-5-[2-(diäthylamino)äthyl]-2,3-dihydro-2-(4-methoxyphenyl)naphtho[2,1-b][1,4]thiazepin-4(5H)-on.

13. Verbindungen der allgemeinen Formel

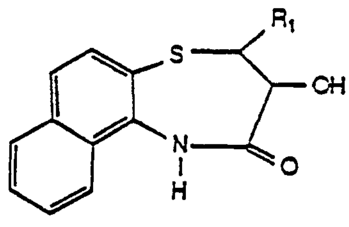

II

worin $R_1$ Phenyl mit 1-3 Niederalkoxy- oder Halogensubstituenten bedeutet.

14. Eine Verbindung gemäss einem der Ansprüche 1-12 zur Anwendung als therapeutischer Wirkstoff.

15. Eine Verbindung gemäss einem der Ansprüche 1-12 zur Anwendung bei der Behandlung oder Verhütung von Ischämie und Bluthochdruck.

16. Verfahren zur Herstellung einer Verbindung gemäss einem der Ansprüche 1-12, dadurch gekennzeichnet, dass man

a) eine Verbindung der allgemeinen Formel

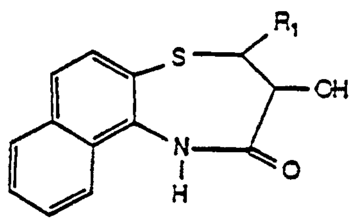

II

worin $R_1$ die in Anspruch 1 angegebene Bedeutung besitzt,
mit einer Verbindung der allgemeinen Formel

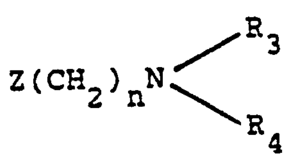

III

worin $R_3$, $R_4$ und n die in Anspruch 1 angegebene Bedeutung besitzen und Z Halogen bedeutet, umsetzt, und erwünschtenfalls,

b) ein Racemat einer erhaltenen Verbindung der allgemeinen Formel

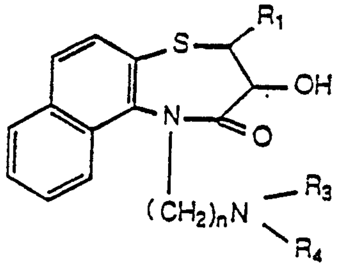

Ie

worin $R_1$, $R_3$, $R_4$ und n die in Anspruch 1 angegebene Bedeutung besitzen, in die optisch aktiven Enantiomeren auftrennt, und/oder

c) ein Alkalimetallsalz einer Verbindung der Formel Ie mit einem Niederalkylierungsmittel umsetzt, oder

d) eine Verbindung der Formel Ie, mit einem Niederalkansäureanhydrid, einem Niederalkanoylhalogenid, einem $C_1$-$C_5$-Alkylhaloformat, einem $C_1$-$C_3$-Alkoxyacetyl- oder -propionylhalogenid oder einem Niedercycloalkylcarbonsäurehalogenid umsetzt, und/oder

e) eine erhaltene Verbindung der Formel I in ein pharmazeutisch verwendbares Säureadditionssalz überführt.

17. Arzneimittel enthaltend eine Verbindung gemäss einem der Ansprüche 1-12 und ein therapeutisch inertes Excipiens.

18. Arzneimittel zur Behandlung oder Verhütung von Ischämie und Bluthochdruck enthaltend eine Verbindung gemäss einem der Ansprüche 1-12 und ein therapeutisch inertes Excipiens.

19. Verwendung einer Verbindung gemäss einem der Ansprüche 1-12 zur Herstellung eines Arzneimittels gegen Ischämie und/oder Bluthochdruck.

Europäisches Patentamt

# EUROPÄISCHER RECHERCHENBERICHT

Nummer der Anmeldung

EP 88 12 0616

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 234 561 (HOFFMANN-LA ROCHE) * Insgesamt * --- | 1-19 | C 07 D 281/08<br>A 61 K 31/55 |
| E | EP-A-0 302 379 (TANABE SEIYAKU) * Insgesamt * ----- | 1-19 | |

| RECHERCHIERTE SACHGEBIETE (Int. Cl.4) |
|---|
| C 07 D 281/00 |

Der vorliegende Recherchenbericht wurde für alle Patentansprüche erstellt

| Recherchenort | Abschlußdatum der Recherche | Prüfer |
|---|---|---|
| DEN HAAG | 14-03-1989 | ALLARD M.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)